(19) Europäisches Patentamt / European Patent Office / Office européen des brevets

(11) EP 2 788 335 B1

(12) # EUROPEAN PATENT SPECIFICATION

(45) Date of publication and mention of the grant of the patent:
**13.04.2016 Bulletin 2016/15**

(21) Application number: **12794730.7**

(22) Date of filing: **04.12.2012**

(51) Int Cl.:
***C07D 265/30*** (2006.01) ***C07D 413/12*** (2006.01)
***A61K 31/5377*** (2006.01) ***A61P 25/00*** (2006.01)

(86) International application number:
**PCT/EP2012/074349**

(87) International publication number:
**WO 2013/083556 (13.06.2013 Gazette 2013/24)**

(54) **5-(3-AMINOPHENYL)-5-ALKYL-5,6-DIHYDRO-2H-[1,4]OXAZIN-3-AMINE DERIVATIVES FOR THE TREATMENT OF DISORDERS IN WHICH BETA-SECRETASE IS INVOLVED**

5-(3-AMINOPHENYL)-5-ALKYL-5,6-DIHYDRO-2H-[1,4]OXAZIN-3-AMIN-DERIVATE ZU BEHANDLUNG VON STÖRUNGEN DIE BETA-SEKRETASE INVOLVIEREN

DÉRIVÉS DE 5-(3-AMINOPHÉNYL)-5-ALKYL-5,6-DIHYDRO-2H-[1,4]OXAZIN-3-AMINE POUR LE TRAITEMENT DE TROUBLES IMPLIQUANT LA BETA-SECRETASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.12.2011 EP 11192171**

(43) Date of publication of application:
**15.10.2014 Bulletin 2014/42**

(73) Proprietor: **Janssen Pharmaceutica, N.V.**
**2340 Beerse (BE)**

(72) Inventors:
- **GIJSEN, Henricus, Jacobus, Maria**
  **B-2340 Beerse (BE)**
- **VAN BRANDT, Sven, Franciscus, Anna**
  **B-2340 Beerse (BE)**

(74) Representative: **Quaghebeur, Luc**
**Janssen Pharmaceutica N.V.**
**Turnhoutseweg 30**
**B-2340 Beerse (BE)**

(56) References cited:
**WO-A1-2011/009943**
**WO-A1-2011/020806**
**WO-A1-2011/071135**
**WO-A1-2011/154431**
**WO-A1-2012/098064**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 788 335 B1

## Description

## FIELD OF THE INVENTION

The present invention relates to novel 5-(3-aminophenyl)-5-alkyl-5,6-dihydro-2*H*-[1,4]oxazin-3-amine derivatives as inhibitors of beta-secretase, also known as beta-site amyloid cleaving enzyme, BACE, BACE1, Asp2, or memapsin2. The invention is also directed to pharmaceutical compositions comprising such compounds, to processes for preparing such compounds and compositions, and to such compounds and compositions for use in the prevention and treatment of disorders in which beta-secretase is involved, such as Alzheimer's disease (AD), mild cognitive impairment, senility, dementia, dementia with Lewy bodies, cerebral amyloid angiopathy, multi-infarct dementia, Down's syndrome, dementia associated with stroke, dementia associated with Parkinson's disease and dementia associated with beta-amyloid.

## BACKGROUND OF THE INVENTION

Alzheimer's Disease (AD) is a neurodegenerative disease associated with aging. AD patients suffer from cognition deficits and memory loss as well as behavioral problems such as anxiety. Over 90% of those afflicted with AD have a sporadic form of the disorder while less than 10% of the cases are familial or hereditary. In the United States, about 1 in 10 people at age 65 have AD while at age 85, 1 out of every two individuals are affected with AD. The average life expectancy from the initial diagnosis is 7-10 years, and AD patients require extensive care either in an assisted living facility which is very costly or by family members. With the increasing number of elderly in the population, AD is a growing medical concern. Currently available therapies for AD merely treat the symptoms of the disease and include acetylcholinesterase inhibitors to improve cognitive properties as well as anxiolytics and antipsychotics to control the behavioral problems associated with this ailment.

The hallmark pathological features in the brain of AD patients are neurofibillary tangles which are generated by hyperphosphorylation of tau protein and amyloid plaques which form by aggregation of beta-amyloid 1-42 (Abeta 1-42) peptide. Abeta 1-42 forms oligomers and then fibrils, and ultimately amyloid plaques. The oligomers and fibrils are believed to be especially neurotoxic and may cause most of the neurological damage associated with AD. Agents that prevent the formation of Abeta 1-42 have the potential to be disease-modifying agents for the treatment of AD. Abeta 1-42 is generated from the amyloid precursor protein (APP), comprised of 770 amino acids. The N-terminus of Abeta 1-42 is cleaved by beta-secretase (BACE), and then gamma-secretase cleaves the C-terminal end. In addition to Abeta 1-42, gamma-secretase also liberates Abeta 1-40 which is the predominant cleavage product as well as Abeta 1-38 and Abeta 1-43. These Abeta forms can also aggregate to form oligomers and fibrils. Thus, inhibitors of BACE would be expected to prevent the formation of Abeta 1-42 as well as Abeta 1-40, Abeta 1-38 and Abeta 1-43 and would be potential therapeutic agents in the treatment of AD.

WO-2011/009943 (Novartis) discloses unsubstituted and 2-substituted oxazine derivatives and their use as BACE inhibitors for the treatment of neurological disorders. WO-2011/020806 (Hoffmann-LaRoche) discloses 2,6-unsubstituted 3-amino-5-phenyl-5,6-dihydro-2H-[1,4]oxazine derivatives having BACE1 and /or BACE2 inhibitory properties.

## SUMMARY OF THE INVENTION

The present invention is directed to 5,6-dihydro-2*H*-[1,4]oxazin-3-ylamine derivatives of Formula (I)

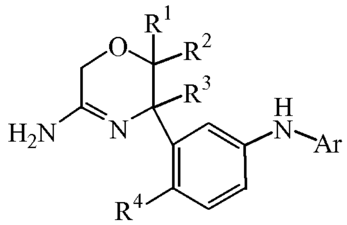

and the tautomers and the stereoisomeric forms thereof, wherein

$R^1$ is fluoro, fluoromethyl, difluoromethyl or trifluoromethyl;
$R^2$ is hydrogen or trifluoromethyl; or
$R^1$ and $R^2$ form a divalent radical $=CF_2$;

$R^3$ is hydrogen, $C_{1-3}$alkyl, cyclopropyl, mono- or polyhalo-$C_{1-3}$alkyl;
$R^4$ is hydrogen or fluoro;
Ar is homoaryl or heteroaryl;
wherein homoaryl is phenyl or phenyl substituted with one, two or three substituents selected from the group consisting of halo, cyano, $C_{1-3}$alkyl, $C_{1-3}$alkyloxy, mono- and polyhalo-$C_{1-3}$alkyl, mono-and polyhalo-$C_{1-3}$alkyloxy;
heteroaryl is selected from the group consisting of pyridyl, pyrimidyl, pyrazyl, pyridazyl, furanyl, thienyl, pyrrolyl, pyrazolyl, imidazolyl, triazolyl, tetrazolyl, thiazolyl, isothiazolyl, thiadiazolyl, oxazolyl, isoxazolyl, and oxadiazolyl, each optionally substituted with one, two or three substituents selected from the group consisting of halo, cyano, $C_{1-3}$alkyl, $C_{2-3}$alkynyl, $C_{1-3}$alkyloxy, mono- and polyhalo-$C_{1-3}$alkyl, mono- and polyhalo-$C_{1-3}$alkyloxy, and $C_{1-3}$alkyloxy$C_{1-3}$alkyloxy; and the addition salts thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

Also disclosed are methods of treating a disorder mediated by the beta-secretase enzyme, comprising administering to a subject in need thereof a therapeutically effective amount of any of the compounds or pharmaceutical compositions described above.

Also disclosed are methods of inhibiting the beta-secretase enzyme, comprising administering to a subject in need thereof a therapeutically effective amount of any of the compounds or pharmaceutical compositions described above.

Also disclosed is a method of treating a disorder selected from the group consisting of Alzheimer's disease, mild cognitive impairment, senility, dementia, dementia with Lewy bodies, cerebral amyloid angiopathy, multi-infarct dementia, Down's syndrome, dementia associated with stroke, dementia associated with Parkinson's disease and dementia associated with beta-amyloid, preferably Alzheimer's disease, comprising administering to a subject in need thereof, a therapeutically effective amount of any of the compounds or pharmaceutical compositions described above.

Another example of the invention is any of the compounds described above for use in treating: (a) Alzheimer's Disease, (b) mild cognitive impairment, (c) senility, (d) dementia, (e) dementia with Lewy bodies, (f) Down's syndrome, (g) dementia associated with stroke, (h) dementia associated with Parkinson's disease and (i) dementia associated with beta-amyloid, in a subject in need thereof.

## DETAILED DESCRIPTION OF THE INVENTION

The present invention is directed to compounds of formula (I) as defined hereinbefore, and pharmaceutically acceptable salts and solvates thereof. The compounds of formula (I) are inhibitors of the beta-secretase enzyme (also known as beta-site cleaving enzyme, BACE, BACE1 , Asp2 or memapsin 2), and are useful in the treatment of Alzheimer's disease, mild cognitive impairment, senility, dementia, dementia associated with stroke, dementia with Lewy bodies, Down's syndrome, dementia associated with Parkinson's disease and dementia associated with beta-amyloid, preferably Alzheimer's disease, mild cognitive impairment or dementia, more preferably Alzheimer's disease.

In an embodiment of the invention,

$R^1$ is fluoro, difluoromethyl or trifluoromethyl;
$R^2$ is hydrogen or trifluoromethyl; or
$R^1$ and $R^2$ form a divalent radical $=CF_2$;
$R^3$ is $C_{1-3}$alkyl, cyclopropyl, mono- or polyhalo-$C_{1-3}$alkyl;
$R^4$ is hydrogen or fluoro;
Ar is heteroaryl;
wherein heteroaryl is pyridyl or pyrimidyl, each optionally substituted with one, two or three substituents selected from the group consisting of halo, cyano, $C_{1-3}$alkyl, $C_{2-3}$alkynyl, $C_{1-3}$alkyloxy, mono- and polyhalo-$C_{1-3}$alkyl, mono- and polyhalo-$C_{1-3}$alkyloxy, and $C_{1-3}$alkyloxy$C_{1-3}$alkyloxy;

and the addition salts and the solvates thereof

In another embodiment,

$R^1$ is fluoro, difluoromethyl or trifluoromethyl, and $R^2$ is hydrogen; or
$R^1$ is fluoro and $R^2$ is trifluoromethyl; or
$R^1$ and $R^2$ form a divalent radical $=CF_2$;

$R^3$ is methyl or cyclopropyl;
$R^4$ is hydrogen or fluoro;
Ar is heteroaryl;
wherein heteroaryl is pyridyl substituted with methoxy, or pyrimidyl;

and the addition salts and the solvates thereof

In another embodiment the carbon atom substituted with $R^3$ has the R configuration.

In another embodiment the compound of formula (I) is selected from

(5*R*,6*S*)-5-cyclopropyl-6-fluoro-5-{3-[(3-methoxypyridin-2-yl)amino]phenyl}-6-(trifluoromethyl)-5,6-dihydro-2H-1,4-oxazin-3-amine;
(5*R*,6*R*)-6-fluoro-5-{2-fluoro-5-[(3-methoxypyridin-2-yl)amino]phenyl}-5-methyl-5,6-dihydro-2H-1,4-oxazin-3-amine,
*(5R)*-6-(difluoromethylidene)-5-{2-fluoro-5-[(3-methoxypyridin-2-yl)amino]phenyl}-5-methyl-5,6-dihydro-2H-1,4-oxazin-3-amine;
(5*R*,6*R**)-6-(Difluoromethyl)-5-{2-fluoro-5-[(3-methoxypyridin-2-yl)amino]phenyl}-5-methyl-5,6-dihydro-2H-1,4-oxazin-3-amine;
(5*R*,6*R*)-6-fluoro-5-[2-fluoro-5-(pyrimidin-2-ylamino)phenyl]-5-methyl-5,6-dihydro-2H-1,4-oxazin-3-amine; (5*R*,6*R*)-5-{2-fluoro-5-[(3-methoxypyridin-2-yl)amino]phenyl}-5-methyl-6-(trifluoromethyl)-5,6-dihydro-2H-1,4-oxazin-3-amine;
(5*R*,6*R*)-5-[2-fluoro-5-(pyrimidin-2-ylamino)phenyl]-5-methyl-6-(trifluoromethyl)-5,6-dihydro-2H-1,4-oxazin-3-amine;
(5*R*,6*R*)-6-fluoro-5-{2-fluoro-5-[(3-methoxypyrazin-2-yl)amino]phenyl}-5-methyl-5,6-dihydro-2*H*-1,4-oxazin-3-amine; and
(5*R*,6*R*)-5-{2-fluoro-5-[(3-methoxypyrazin-2-yl)amino]phenyl}-5-methyl-6-(trifluoromethyl)-5,6-dihydro-2*H*-1,4-oxazin-3-amine.

## DEFINITIONS

"Halo" shall denote fluoro, chloro and bromo; "$C_{1-3}$alkyl" shall denote a straight or branched saturated alkyl group having 1, 2 or 3 carbon atoms, e.g. methyl, ethyl, 1-propyl and 2-propyl; "$C_{1-3}$alkyloxy" shall denote an ether radical wherein $C_{1-3}$alkyl is as defined before; "mono- and polyhalo$C_{1-3}$alkyl" shall denote $C_{1-3}$alkyl as defined before, substituted with 1, 2, 3 or where possible with more halo atoms as defined before; "mono- and polyhalo$C_{1-3}$alkyloxy" shall denote an ether radical wherein mono-and polyhalo$C_{1-3}$alkyl is as defined before; "$C_{3-6}$cycloalkyl" shall denote cyclopropyl, cyclobutyl, cyclopentyl and cyclohexyl; "$C_{3-6}$cycloalkanediyl" shall denote a bivalent radical such as cyclopropanediyl, cyclobutanediyl, cyclopentanediyl and cyclohexanediyl.

The term "subject" as used herein, refers to an animal, preferably a mammal, most preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

Hereinbefore and hereinafter, the term "compound of formula (I)" is meant to include the addition salts, the solvates and the stereoisomers thereof.

The terms "stereoisomers" or "stereochemically isomeric forms" hereinbefore or hereinafter are used interchangeably.

The invention includes all stereoisomers of the compound of Formula (I) either as a pure stereoisomer or as a mixture of two or more stereoisomers.

Enantiomers are stereoisomers that are non-superimposable mirror images of each other. A 1:1 mixture of a pair of enantiomers is a racemate or racemic mixture. Diastereomers (or diastereoisomers) are stereoisomers that are not enantiomers, i.e. they are not related as mirror images. If a compound contains a double bond, the substituents may be in the E or the Z configuration. If a compound contains a disubstituted cycloalkyl group, the substituents may be in the cis or trans configuration. Therefore, the invention includes enantiomers, diastereomers, racemates, E isomers, Z isomers, cis isomers, trans isomers and mixtures thereof.

The absolute configuration is specified according to the Cahn-Ingold-Prelog system. The configuration at an

asymmetric atom is specified by either R or S. Resolved compounds whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) is for instance specified as (R), this means that the compound is substantially free of the (S) isomer; when a compound of formula (I) is for instance specified as E, this means that the compound is substantially free of the Z isomer; when a compound of formula (I) is for instance specified as cis, this means that the compound is substantially free of the trans isomer.

The compounds of Formula (I) co-exist in a dynamic equilibrium with the tautomers of Formula (I-a).

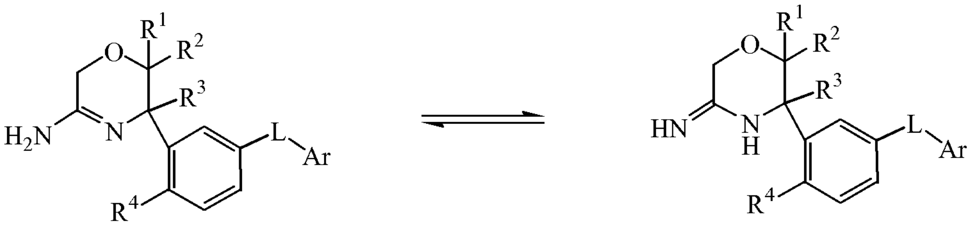

(I) (I-a)

For use in medicine, the salts of the compounds of this invention refer to nontoxic "pharmaceutically acceptable salts". Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

Representative acids which may be used in the preparation of pharmaceutically acceptable salts include, but are not limited to, the following: acetic acid, 2,2-dichloroactic acid, acylated amino acids, adipic acid, alginic acid, ascorbic acid, L-aspartic acid, benzenesulfonic acid, benzoic acid, 4- acetamidobenzoic acid, (+)-camphoric acid, camphorsulfonic acid, capric acid, caproic acid, caprylic acid, cinnamic acid, citric acid, cyclamic acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucoronic acid, L-glutamic acid, beta-oxo-glutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5- disulfonic acid, 1-hydroxy-2-naphthoic acid, nicotinic acid, nitric acid, oleic acid, orotic acid, oxalic acid, palmitic acid, pamoic acid, phosphoric acid, L- pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, trifluoromethylsulfonic acid, and undecylenic acid. Representative bases which may be used in the preparation of pharmaceutically acceptable salts include, but are not limited to, the following: ammonia, L-arginine, benethamine, benzathine, calcium hydroxide, choline, dimethylethanolamine, diethanolamine, diethylamine, 2-(diethylamino)-ethanol, ethanolamine, ethylenediamine, *N*-methyl-glucamine, hydrabamine, 1*H*-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The names of the compounds of the present invention were generated according to the nomenclature rules agreed upon by the Chemical Abstracts Service (CAS) using Advanced Chemical Development, Inc., software (ACD/Name product version 10.01; Build 15494, 1 Dec 2006) or according to the nomenclature rules agreed upon by the International Union of Pure and Applied Chemistry (IUPAC) using Advanced Chemical Development, Inc., software (ACD/Name product version 10.01.0.14105, October 2006). In case of tautomeric forms, the name of the depicted tautomeric form of the structure was generated. The other non-depicted tautomeric form is also included within the scope of the present invention.

## Preparation of the compounds

Experimental procedure 1

The final compounds according to Formula (I), can be prepared by reacting an intermediate compound of Formula (II) with a compound of Formula (III) according to reaction scheme (1), a reaction that is performed in a suitable reaction-inert solvent, such as, for example, isopropanol or 1,4-dioxane, in the presence of a suitable acid, such as, for example, $H_2SO_4$ or HCl, under thermal conditions such as, for example, heating the reaction mixture at 100 °C, for example for 16 hours.This conversion can also be performed in the presence of a Pd-complex catalyst such as, for example, tris(dibenzylideneacetone)dipalladium(0) [CAS 51364-51-3] in a suitable reaction-inert solvent, such as, for example, 1,4-dioxane, ethanol or mixtures of inert solvents, in the presence of a suitable base, such as, for example, aqueous $K_3PO_4$, $Na_2CO_3$ or $Cs_2CO_3$ and a suitable ligand such as, for example, 1,1'-bis(diphenylphosphino)-ferrocene [CAS 12150-46-8], under thermal conditions such as, for example, heating the reaction mixture at 160 °C under microwave irradiation until completion of the reaction, for example 1 hour. In reaction scheme (1), all variables are defined as in Formula (I) and W is halo.

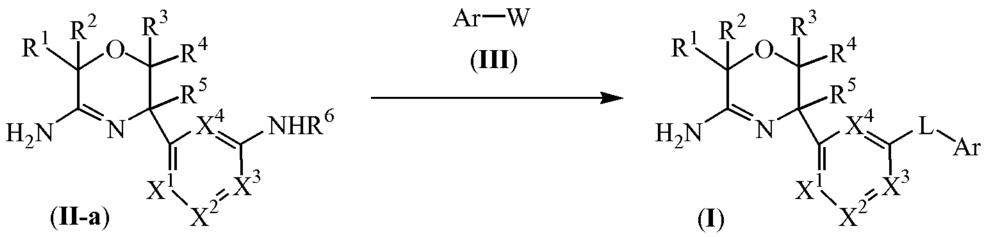

(II-a) (I)

Reaction Scheme 1

Experimental procedure 2

The final compounds according to Formula (I-b) wherein $R^3$ is difluoromethyl and $R^4$ is an hydrogen, can be prepared by catalytic hydrogenation of an intermediate compound of Formula (I-a) according to reaction scheme (2). Said conversion may be conducted by treatment of the intermediate compound of Formula (I-a) with hydrogen in the presence of a suitable catalyst such as, for example, palladium on carbon, a suitable catalyst poison, such as, for example, thiophene, in a suitable reaction-inert solvent, such as, for example, ethyl acetate. The mixture is stirred under hydrogen atmosphere, at a suitable temperature, typically room temperature, at a suitable pressure, such as, for example, atmospheric pressure, for example for 16 hours. In reaction scheme (2), all variables are defined as in Formula (I).

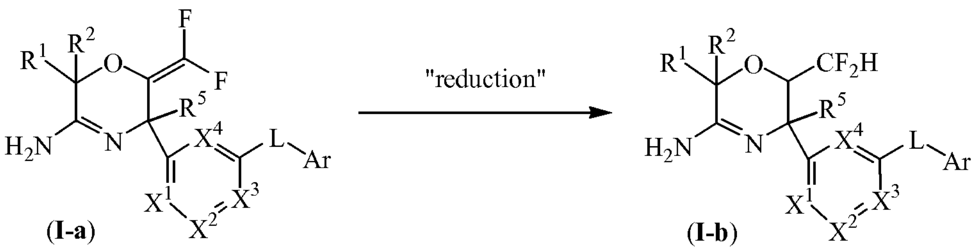

(I-a) (I-b)

Reaction Scheme 2

Experimental procedure 3

The intermediate compounds of Formula (II-a) and (II-b) can generally be prepared following the reaction steps shown in the reaction scheme (3) below.

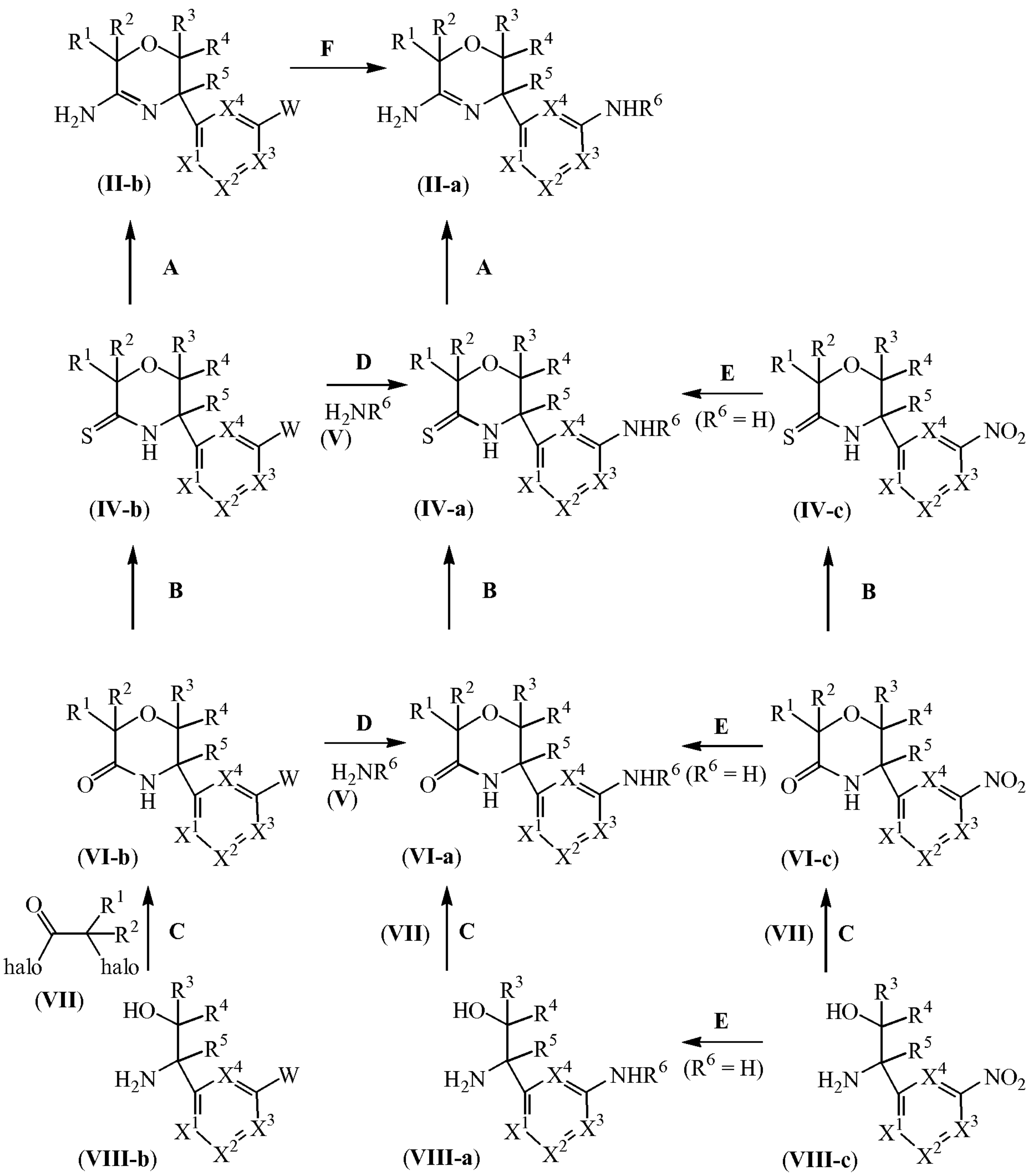


Reaction Scheme 3

**A**: thioamide-to-amidine conversion
**B**: amide-to-thioamide conversion (thionation)
**C**: cyclization
**D**: Buchwald-Hartwig type coupling (when W is Halo)
**E**: nitro-to-amino reduction (when $R^6$ is H)
**F**: Bromo-to-amine conversion (when $R^6$ is H)

The amidine derivatives in the above reaction scheme may be conveniently prepared from the corresponding thioamide derivatives following art-known thioamide-to-amidine conversion procedures (reaction step A). Said conversion may conveniently be conducted by treatment of the said thioamides with an ammonia source such as, for example, ammonium chloride or aqueous ammonia, in a suitable reaction-inert solvent such as, for example, water or methanol and the like, under thermal conditions such as, for example, heating the reaction mixture at 60 °C, for example for 6 hours.

Alternatively, intermediate compounds of Formula (II-a) wherein $R^6$ is hydrogen in the above reaction scheme (3) can be prepared from the corresponding intermediate compounds of Formula (II-b) via copper catalyzed type coupling procedure (reaction step F). Said coupling may be conducted by treatment of said intermediate compounds of Formula (II-b) with sodium azide in a suitable reaction-inert solvent, such as, for example, DMSO, in the presence of a mixture of suitable bases, such as, for example, dimethylethylenediamine and $Na_2CO_3$, and a copper catalyst such as, CuI, under thermal conditions such as, for example, heating the reaction mixture at 110 °C, until completion of the reaction, for example 1 hour.

The thioamide derivatives in the above reaction scheme (3) can be prepared from amide derivatives following art-known thionation procedures (reaction step B). Said conversion may conveniently be conducted by treatment of the said amides with a thionation agent such as, for example, phosphorous pentasulfide or 2,4-bis-(4-methoxy-phenyl)-1,3-dithia-2,4-diphosphetane 2,4-disulfide [Lawesson's reagent, CAS 19172-47-5], in a reaction inert solvent such as, for example, tetrahydrofuran or 1,4-dioxane and the like, under thermal conditions such as, for example, heating the reaction mixture at 50 °C, for example for 50 minutes.

The amide derivatives in the above reaction scheme (3) can be prepared from the beta-aminoalcohol derivatives of Formula (VIII) and intermediate compounds of Formula (VII) following art-known cyclization procedures (reaction step C). Said cyclization may conveniently be conducted by treatment of the said beta-aminoalcohols with an intermediate compound of Formula (VII) in the presence of a base, such as potassium *tert*-butoxide, or a mixture of bases such as potassium *tert*-butoxide/*N,N*-diisopropylethylamine a reaction inert solvent, such as for example tetrahydrofuran and the like, at -80 °C to 100 °C, preferably -15 °C to 25 °C for 30 minutes to 100 hours, preferably 1 hour to 24 hours.

Additionally intermediate compounds of Formula (IV-a) and (VI-a) in the above reaction scheme (3) can be prepared from the corresponding intermediate compounds of Formula (IV-b) and (VI-b) following art-known Buchwald-Hartwig type coupling procedures (reaction step D). Said coupling may be conducted by treatment of intermediate compounds of Formula (IV-b) and (VI-b) with an intermediate compound of Formula (V) in a suitable reaction-inert solvent, such as, for example, ethanol or mixtures of inert solvents such as, for example, 1,2-dimethoxyethane/water/ethanol, in the presence of a suitable base, such as, for example, aqueous $K_3PO_4$ or $Cs_2CO_3$, a Pd-complex catalyst such as, for example, [1,1'-bis(diphenylphosphino)ferrocene]-dichloropalladium(II) [CAS 72287-26-4] or *trans*-bis(dicyclohexylamine)palladium diacetate [DAPCy, CAS 628339-96-8] under thermal conditions such as, for example, heating the reaction mixture at 80 °C, for example for 20 hours or for example , heating the reaction mixture at 130 °C, for example for 10 minutes under microwave irradiation.

Additionally intermediate compounds of Formula (IV-a) and (VI-a) in the above reaction scheme (3), wherein $R^6$ = H, can be prepared from the corresponding intermediate compounds of Formula (IV-c) and (VI-c) following art-known nitro-to-amino reduction procedures (reaction step E). Said reduction may conveniently be conducted following art-known catalytic hydrogenation procedures. For example, said reduction may be carried out by stirring the reactants under a hydrogen atmosphere and in the presence of an appropriate catalyst such as, for example, palladium-on-charcoal, platinum-on-charcoal, Raney-nickel and the like catalysts. Suitable solvents are, for example, water, alkanols, e.g. methanol, ethanol and the like, esters, e.g. ethyl acetate and the like. In order to enhance the rate of said reduction reaction it may be advantageous to elevate the temperature and/or the pressure of the reaction mixture. Undesired further hydrogenation of certain functional groups in the reactants and the reaction products may be prevented by the addition of a catalyst poison such as, for example, thiophene and the like, to the reaction mixture.

The intermediate compounds of Formula (VII), (VIII-a), (VIII-b) and (VIII-c) can generally be prepared following art-known Strecker type procedures described in literature, followed by standard chemical transformations of the cyano group.

Experimental procedure 4

The intermediate compounds of Formula (IX-a), (IX-b) and (IX-c) can generally be prepared following the reaction steps shown in the reaction scheme (4) below.

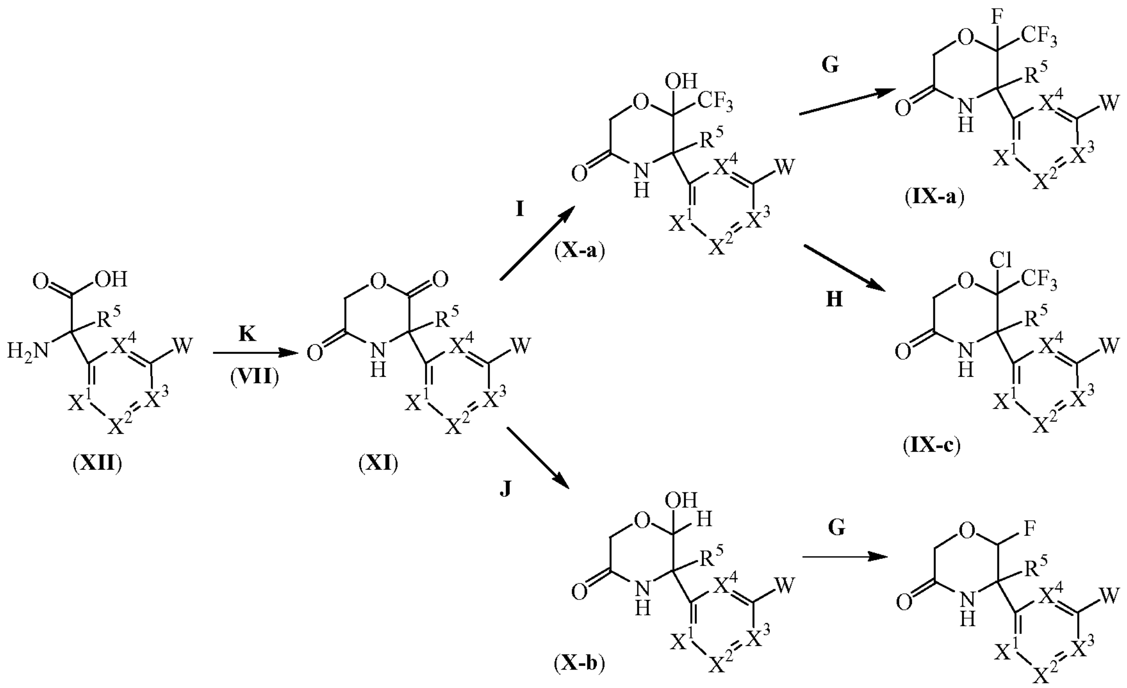


(XII) (XI) (X-a) (X-b) (IX-a) (IX-c) (IX-b)

Reaction Scheme 4

**G:** fluorination
**H:** chlorination
**I:** trifluoromethylation
**J:** reduction
**K:** cyclization

Intermediate compounds of Formula (IX-a) and (IX -b) in the above reaction scheme (4) can be prepared from an intermediate compound of Formula (X-a) and (X -b) following art-known fluorination procedures (reaction step G). Said conversion may be conducted by treatment of the intermediate compounds of Formula (X-a) and (X -b) in the presence of a fluorinating agent such as for example diethylaminosulphur trifluoride (DAST) in a suitable reaction inert solvent, such as for example dichloromethane. The reaction mixture is stirred at suitable temperature, for example 0° C for the required time to achieve completion of the reaction, for example 20-40 minutes.

Intermediate compound of Formula (IX-c) in the above reaction scheme (4) can be prepared from intermediate compounds of Formula (X-a) following art-known chlorination procedures (reaction step H). Said conversion may be conducted by treatment of the intermediate compound of Formula (X-a) with a suitable chlorinating agent such as, for example, thionyl chloride, in the presence of a base such as, for example, pyridine in a reaction-inert solvent, such as, for example, dichloromethane. The reaction mixture is stirred at suitable temperature, for example 0 °C for the required time to achieve completion of the reaction, for example 30-60 minutes.

Intermediate compounds of Formula (X-a) of the above reaction scheme (4) can be prepared from intermediate compounds of Formula (XI) following art-known trifluoromethylation procedures (reaction step I). Said conversion may be conducted by treatment of the intermediate compound of Formula (XI) in the presence of tetrabutyl ammonium fluoride (TBAF), with a trifluoromethylating agent such as, for example, (trifluoromethyl)trimethyl silane, in a suitable reaction-inert solvent, such as, for example, tetrahydrofuran. The reaction mixture is stirred at suitable temperature, for example room temperature for the required time to achieve completion of the reaction, for example two hours.

Intermediate compounds of Formula (X-b) in the above reaction scheme (4) can be prepared from intermediate compounds of Formula (XI) following art-known reduction procedures (reaction step J). Said conversion may be conducted by treatment of the intermediate compound of Formula (XI) with a reducing agent such as, for example, diisobutylaluminium hydride, in a suitable reaction-inert solvent, such as for example tetrahydrofuran. The reaction mixture is stirred at suitable temperature, typically from -78° C to room temperature for the required time to achieve completion of the reaction, for example two hours.

Intermediate compounds of Formula (XI) in the above reaction scheme (4) can be prepared from intermediate compounds of Formula (XII) following art-known two-step cyclization procedures (reaction step K). Said conversion may

be conducted by first, treatment of the intermediate compounds of Formula (XII) with an intermediate compound of Formula (VII), such as, for example, chloroacetylchloride in the presence of a base such as, for example, NaOH, in a suitable mixture of inert solvents such as, for example, water and 1,4-dioxane or water and THF. The pH of the reaction mixture is adjusted to a suitable pH value, for example, 10-11, by addition of a suitable base such as, for example, NaOH. The reaction mixture is stirred at a suitable temperature, for example, 0 °C to 25 °C for the required time to achieve completion of the reaction, for example 1-4 hours. The obtained crude residue can subsequently be cyclised to provide the intermediate (XI) by the addition of a suitable base such as, for example, $K_2CO_3$, $Cs_2CO_3$, *N,N*-diisopropylethylamine or $NaHCO_3$, in a suitable reaction-inert solvent, such as for example, acetonitrile or DMF. The reaction mixture is stirred under thermal conditions such as, for example, heating the reaction mixture at 25 °C to 80°C for 2-24 hours or for example, heating the reaction mixture at 140 °C for 15-30 minutes under microwave irradiation. This conversion can also be performed in the absence of a base in a suitable reaction-inert solvent, such as for example, acetonitrile or DMF, at a suitable temperature, typically 40 °C to 110 °C, for a period of, for example, 24-48 hours.

Experimental procedure 5

The intermediate compounds of Formula (XIII) and (XIV) can generally be prepared from intermediate compounds of Formula (IX-c) following art-known reductive dehalogenation procedures (reaction step L). Said conversion may be conducted by treatment of the intermediate of Formula (IX-c) with a suitable zinc reagent, such as, for example, zinc dust or zinc copper couple in a suitable solvent, such as acetic acid, at a suitable temperature, typically from room temperature to 80 °C, for the required time to achieve completion of the reaction, for example 1-16 hours. This conversion affords a mixture of the intermediate compounds of Formula (XIII) and (XIV) in different ratio depending on the reaction conditions and the reactants.

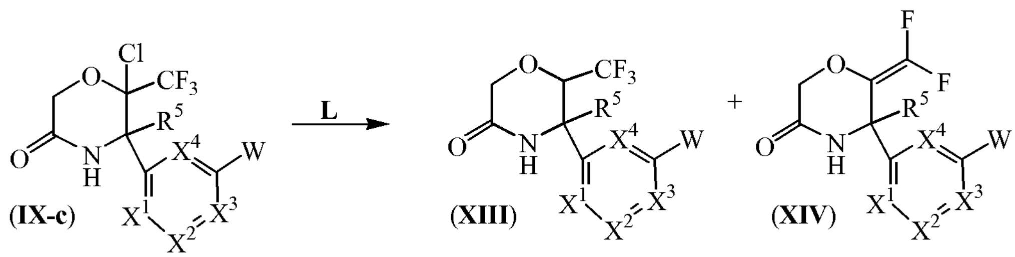


Reaction Scheme 5

**PHARMACEUTICAL COMPOSITIONS**

The present invention also provides compositions for preventing or treating diseases in which inhibition of beta-secretase is beneficial, such as Alzheimer's disease (AD), mild cognitive impairment, senility, dementia, dementia with Lewy bodies, Down's syndrome, dementia associated with stroke, dementia associated with Parkinson's disease and dementia associated with beta-amyloid. Said compositions comprising a therapeutically effective amount of a compound according to formula (I) and a pharmaceutically acceptable carrier or diluent.

While it is possible for the active ingredient to be administered alone, it is preferable to present it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of this invention may be prepared by any methods well known in the art of pharmacy. A therapeutically effective amount of the particular compound, in base form or addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical

carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The exact dosage and frequency of administration depends on the particular compound of formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

Depending on the mode of administration, the pharmaceutical composition will comprise from 0.05 to 99 % by weight, preferably from 0.1 to 70 % by weight, more preferably from 0.1 to 50 % by weight of the active ingredient, and, from 1 to 99.95 % by weight, preferably from 30 to 99.9 % by weight, more preferably from 50 to 99.9 % by weight of a pharmaceutically acceptable carrier, all percentages being based on the total weight of the composition.

The present compounds can be used for systemic administration such as oral, percutaneous or parenteral administration; or topical administration such as via inhalation, a nose spray, eye drops or via a cream, gel, shampoo or the like. The compounds are preferably orally administered. The exact dosage and frequency of administration depends on the particular compound according to formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The amount of a compound of Formula (I) that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg of the active compound. A preferred unit dose is between 1 mg to about 500 mg. A more preferred unit dose is between 1 mg to about 300mg. Even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

For the compositions, methods and kits provided above, one of skill in the art will understand that preferred compounds for use in each are those compounds that are noted as preferred above. Still further preferred compounds for the compositions, methods and kits are those compounds provided in the non-limiting Examples below.

## EXPERIMENTAL PART

Hereinafter, the term "m.p." means melting point, "aq." means aqueous, "r.m." means reaction mixture, "r.t." means room temperature, 'DIPEA' means *N,N*-diisopropylethylamine, "DIPE" means diisopropylether, 'THF' means tetrahydrofuran, 'DMF' means dimethylformamide, 'DCM' means dichloromethane, "EtOH" means ethanol 'EtOAc' means ethylacetate, "AcOH" means acetic acid, "iPrOH" means isopropanol, "$iPrNH_2$" means isopropylamine, "MeCN" means acetonitrile, "MeOH" means methanol, "$Pd(OAc)_2$" means palladium(II)diacetate, "rac" means racemic, 'sat.' means saturated, 'SFC' means supercritical fluid chromatography, 'SFC-MS' means supercritical fluid chromatography/mass spectrometry, "LC-MS" means liquid chromatography/mass spectrometry, "GCMS" means gas chromatography/mass spectrometry, "HPLC" means high-performance liquid chromatography, "RP" means reversed phase, "UPLC" means ultra-performance liquid chromatography, "$R_t$" means retention time (in minutes), "$[M+H]^+$" means the protonated mass of the free base of the compound, "DAST" means diethylaminosulfur trifluoride, "DMTMM" means 4-(4,6-dimethoxy-1,3,5-triazin-2-yl)-4-methylmorpholinium chloride, "HATU" means *O*-(7-azabenzotriazol-1-yl)-*N,N,N',N'*-tetramethyluronium hexafluorophosphate, "Xantphos" means. (9,9-dimethyl-9H-xanthene-4,5-diyl)bis[diphenylphosphine], "TBAT" means tetrabutyl ammonium triphenyldifluorosilicate, "TFA" means trifuoroacetic acid, "Et2O" means diethylether, "DMSO" means dimethylsulfoxide, "MeCN" means acetonitrile.

For key intermediates, as well as some final compounds, the absolute configuration of chiral centers (indicated as *R* and/or *S*) were established via comparison with samples of known configuration, or the use of analytical techniques suitable for the determination of absolute configuration, such as VCD (vibrational cicular dichroism) or X-ray crystallography. When the absolute configuration at a chiral center is unknown, it is arbitrarily designated R*.

A. Preparation of the intermediates

Example A1

Preparation of **intermediate 1.**

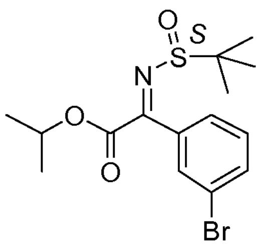

Titanium(IV) isopropoxide (202 mL, 658 mmol) was added to a stirred mixture of ethyl 2-(3-bromo-phenyl)-2-oxo-acetate [(CAS 62123-80-2), 80 g, 329 mmol] and (*S*)-2-methyl-2-propanesulfinamide (47.9 g, 395 mmol) in n-heptane (740 mL). The mixture was stirred at 80 °C for 4 hours. The mixture was cooled to room temperature, and water was added. The resulting mixture was filtered over a diatomaceous earth pad and rinsed with n-heptane. The organic layer was separated, dried ($MgSO_4$), filtered and concentrated *in vacuo.* The residue was purified by flash column chromatography (silica gel; eluent: n-heptane/EtOAc 100/0 to 50/50). The desired fractions were collected and concentrated *in vacuo* to yield **intermediate 1** (91 g, 74% yield).

Example A2

Preparation of **intermediate 2.**

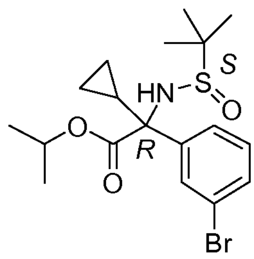

Cyclopropylmagnesium bromide (1 M, 300 mL, 300 mmol) was added dropwise to a stirred solution of **intermediate 1** (91 g, 243 mmol) in DCM (1500 mL) at -40 °C. The mixture was stirred at this temperature for 30 min, and then the reaction was quenched by the addition of a sat. aq. NH4Cl solution, followed by water. The mixture was extracted with DCM. The organic layer was separated, dried ($MgSO_4$), filtered and the solvents evaporated *in vacuo* to yield **intermediate 2** (100 g, 99% yield), which was used as such in the next step.

Example A3

Preparation of **intermediate 3.**

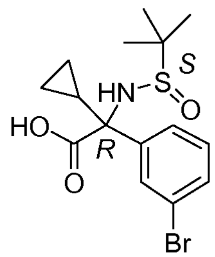

A 1M aq. NaOH solution (750 mL, 750 mmol) was added to a solution of crude **intermediate 2** (100 g, 240 mmol) in MeOH (400 mL). The resulting mixture was stirred at reflux for 4 hours. The mixture was cooled to r.t., and then partitioned between water and EtOAc. The aqueous layer was separated and neutralized by the addition of a 1M aq. HCl solution (750 mL), and then extracted with DCM. The organic layer was separated, dried ($MgSO_4$), filtered and the solvents evaporated in vacuo. The residue was triturated with DIPE/MeCN, and the resulting solids were filtered off and dried in vacuo to yield **intermediate 3** (37 g, 41% yield).
$\alpha_D$: +37.59 ° (589 nm, c 0.564 w/v %, MeOH, 20 °C). The absolute configuration was determined by X-ray diffraction.

Example A4

Preparation of **intermediate 4.**

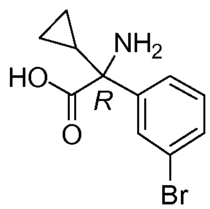

Hydrochloric acid salt

**Intermediate 3** (37 g, 99 mmol) was stirred in 4M HCl solution in dioxane (74 mL) and 1,4-dioxane (75 mL) at room temperature for 30 min. To the resulting suspension, DIPE was added, and the precipitate was filtered off and dried *in vacuo* to yield **intermediate 4** (28 g, 92% yield).
$\alpha_D$: -68.89 ° (589 nm, c 0.646 w/v %, MeOH, 20 °C)

Example A5

Preparation of **intermediate 5.**

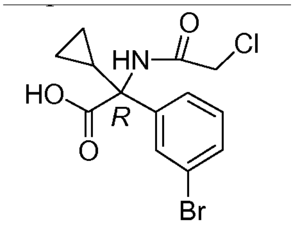

A 1M aq. NaOH solution (182.6 mL, 182.6 mmol) was added to a solution of **intermediate 4** (28 g, 91.3 mmol), and the mixture was cooled on an ice-bath. To this mixture, a solution of chloroacetylchloride (21.8 mL, 274 mmol) in THF (280 mL) was added dropwise at 15 °C over one hour, while simultaneously adding a solution of a 25% aq. NaOH solution to maintain the pH around 10-11. After completion of the reaction, conc. aq. HCl solution was added carefully to the reaction mixture until pH 2. The mixture was partly concentrated *in vacuo,* and the resulting precipitate was filtered off, washed with DIPE, and dried *in vacuo* to give **intermediate 5** (26 g, 82% yield). $\alpha_D$: -6.49 ° (589 nm, c 0.5855 w/v %, MeOH, 20 °C)

Example A6

Preparation of **intermediate 6.**

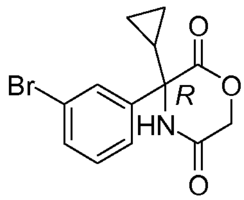

**Intermediate 5** (0.7 g, 2.02 mmol) and $NaHCO_3$ (0.34 g, 4.04 mmol) were dissolved in DMF (17 mL), and the reaction mixture was stirred at 80 °C for 2 hours. The mixture was partially concentrated under reduced pressure, cooled to r.t. and then filtered over diatomaceous earth. The filtrate was concentrated *in vacuo,* and the residue was purified by flash column chromatography (silica gel; eluent: n-heptane/EtOAc 100/0 to 50/50). The desired fractions were collected and concentrated *in vacuo* to yield **intermediate 6** (0.54 g, 86% yield).
$\alpha_D$ : -15.68 ° (589 nm, c 0.37 w/v %, MeOH, 20 °C)

Example A7

Preparation of **intermediate 7**.

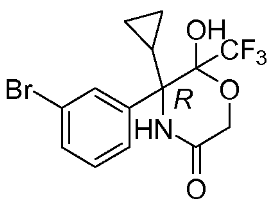

To a solution of **intermediate 6** (4.2 g, 13.54 mmol) in THF (55 mL) was added TBAT (0.73 g, 1.35 mmol). Then, (trifluoromethyl)trimethyl silane (4.0 mL, 27 mmol) was added dropwise, and the r.m. was stirred at room temperature for 2 hours. The mixture was quenched with aqueous NaCl, extracted with EtOAc, the organic phase was separated, dried ($MgSO_4$) and concentrated *in vacuo..* The resulting oil was purified by column chromatography (silica gel; eluent: DCM/EtOAc 100/0 to 0/100). The desired fractions were collected and concentrated *in vacuo* to yield **intermediate 7** (3 g, 58% yield) as a mixture of cis and trans isomers, which was used as such in the next step.

Example A8

Preparation of **intermediate 8.**

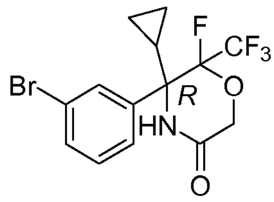

**Intermediate 7** (3 g, 7.9 mmol) was dissolved in DCM (20 mL) and DAST (1.16 mL, 9.5 mmol) was added dropwise at r.t. The reaction mixture was stirred at r.t. for 1 hour and then the r.m. was concentrated under reduced pressure. The residue was partitioned between DCM and an aq. sat. $NaHCO_3$ solution. The organic layer was separated and the aqueous layer was extracted with DCM. The combined organic layers were dried ($MgSO_4$), filtered, and the solvent evaporated *in vacuo.* The residue was purified by flash column chromatography (silica gel; eluent: n-heptane/EtOAc 100/0 to 0/100). The desired fractions were collected and concentrated *in vacuo* to yield **intermediate 8** (2 g, 66% yield) as a mixture of cis and trans isomers, which was used as such in the next step.

Example A9

Preparation of **intermediate 9**.

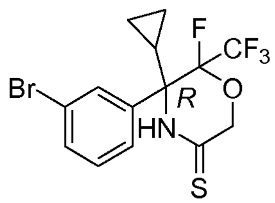

$P_2S_5$ (1.16 g, 5.23 mmol) was added to a solution of **intermediate 8** (2 g, 5.23 mmol) in THF (43 mL) at room temperature. The mixture was stirred at 70 °C for 3 hours. Then the mixture was cooled to room temperature, filtered off and the organic solvent evaporated *in vacuo.* The crude product was purified by flash column chromatography (silica gel; eluent: n-heptane/DCM 80/100 to 0/100). The desired fractions were collected and evaporated *in vacuo* to yield **intermediate 9** (1.6 g, 77% yield) as a mixture of cis and trans isomers.

Example A10

Preparation of **intermediate 10** and **11**.

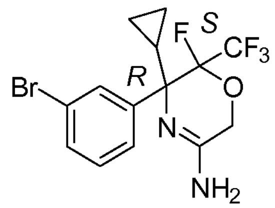

**intermediate 10**

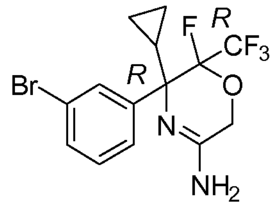

**intermediate 11**

**Intermediate 9** (4.2 g, 10.55 mmol), was added to a mixture of 7N ammonia in MeOH (16 mL) and an aq. $NH_4OH$ solution (40 mL), and the reaction mixture was stirred at 140 °C for 1 hour under microwave irradiation. Then the solvent was evaporated and the residue was dissolved in DCM, dried ($MgSO_4$), filtered, and the solvent evaporated *in vacuo.* The residue was purified by flash column chromatography (silica gel; eluent: n-heptane/EtOAc 100/0 to 50/50). The desired fractions were collected and concentrated *in vacuo* to yield **intermediate 10** (2.44 g, 61% yield) and **intermediate 11** (0.7 g, 17% yield).

Example A11

Preparation of **intermediate 12.**

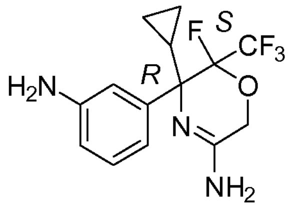

**Intermediate 10** (2.44 g, 6.4 mmol) was combined with $NaN_3$ (1.04 g, 16 mmol), CuI (1.52 g, 8.0 mmol) and $Na_2CO_3$ (1.357 g, 12.8 mmol) in DMSO (92 mL) and the reaction was degassed. After that, *N,N'*-dimethylethylenediamine (1.2 mL, 11.2 mmol) was added and the mixture was heated at 110 °C until completion of the reaction, about 6 hours. The reaction mixture was poured in DCM. Ammonium hydroxide (28% in water) was added and the organic layer was separated and washed three times with ammonium hydroxide solution. Then the organic layer was dried ($Mg_2SO_4$), filtered and concentrated *in vacuo* to yield **intermediate 12** (2 g, 98% yield).

Example A12

Preparation of **intermediate 13.**

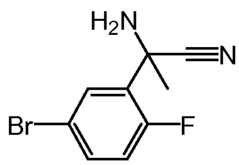

Trimethylsilylcyanide (30.7 mL, 230 mmol) was added to a stirred solution of 5-bromo-2-fluoroacetophenone (25 g, 115 mmol) and $NH_4Cl$ (18.5 g, 345 mmol) in $NH_3$/MeOH (150 mL). The mixture was stirred at room temperature for 3 days. Then the solvent was evaporated *in vacuo* and the residue was taken up in EtOAc (80 mL). The solid was filtered and the filtrate was evaporated *in vacuo* to yield **intermediate 13** (27.9 g, quant. yield) which was used in the next step without further purification.

Example A13

Preparation of **intermediate 14**.

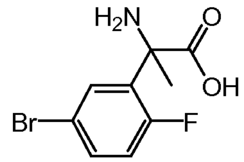

**Intermediate 13** (27 g, 111 mmol) was dissolved in HCl (37% in $H_2O$) (130 mL) and acetic acid (130 mL) and the mixture was refluxed for 16 hours. After cooling to room temperature, the mixture was concentrated *in vacuo.* Water was added and the aqueous layer was extracted with EtOAc. The aqueous layer was basified with aq. NaOH solution (25%) to pH 7. The aqueous layer was partially concentrated *in vacuo.* The mixture was cooled down in an ice bath and the precipitate was filtered off, washed with water and then $Et_2O$ and dried *in vacuo* to yield **intermediate 14** (18 g, 62% yield) as a white solid.

Example A14

Preparation of **intermediate 15**.

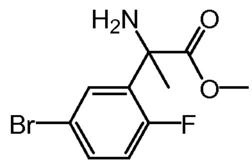

A mixture of **intermediate 14** (15 g, 57.2 mmol) in a solution of 10% $H_2SO_4$ in methanol (330 mL) was refluxed for 48 h. The r.m. was concentrated *in vacuo.* Water was added and the solution was basified to pH 8 with sat. aq. $NHCO_3$ solution. The aqueous layer was then extracted with EtOAc. The organic layer was separated, dried ($MgSO_4$), filtered and concentrated *in vacuo* to yield **intermediate 15** (15 g, 95% yield).

Example A15

Preparation of **intermediate 16.**

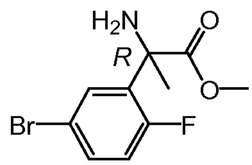

**Intermediate 15** (10 g) was separated into the corresponding enantiomers by preparative SFC on (Chiralpak® Daicel AD 30 x 250 mm). Mobile phase ($CO_2$, MeOH with 0.2% $iPrNH_2$) to yield **intermediate 16** (4.2 g, 42% yield). $\alpha_D$: -10.1 ° (365 nm, c 0.762 w/v %, MeOH, 20 °C).

Example A16

Preparation of **intermediate 17**.

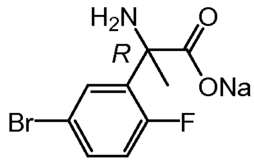

THF (150 mL) was added to a solution of **intermediate 16** (40 g, 145 mmol) in NaOH (1 M in $H_2O$, 360 mL). The mixture was stirred for 4 hours at room temperature. The mixture was concentrated *in vacuo* to afford **intermediate 17** (42 g) as a white solid, which was used as such in the next reaction step.

Example A17

Preparation of **intermediate 18**.

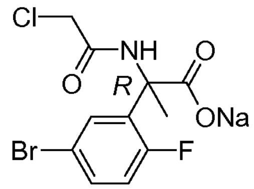

To a cooled solution of **intermediate 17** (41.3 g, 145 mmol) in $H_2O$ (150 mL), a solution of chloroacetyl chloride (24 mL, 304.5 mmol) in 1,4-dioxane (75 mL) was added dropwise. Simultaneously, NaOH (5M in $H_2O$, 29 mL) was added to adjust the pH at 10-11. The organic layer was separated, and the aqueous layer extracted with $Et_2O$. Then the aqueous layer was acidified with HCl (6 M, in $H_2O$) until pH 2. The precipitated white solid was collected by filtration, washed with $H_2O$ and dried to yield **intermediate 18** (42 g, 86% yield).

Example A18

Preparation of **intermediate 19**.

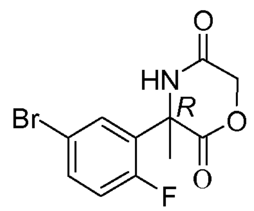

**Intermediate 18** (42 g, 124 mmol) and $NaHCO_3$ (20.8 g, 248 mmol) were dissolved in DMF (1000 mL), and the reaction mixture was stirred at 80 °C for 3 hours. The mixture was partially concentrated under reduced pressure, cooled to r.t. and then filtered over diatomaceous earth. The filtrate was concentrated *in vacuo,* and the residue was purified by flash column chromatography (silica gel; eluent: MeOH/DCM 0/100 to 5/95). The desired fractions were collected and concentrated *in vacuo* to yield **intermediate 19** (36 g, 96% yield).

Example A19

Preparation of **intermediate 20.**

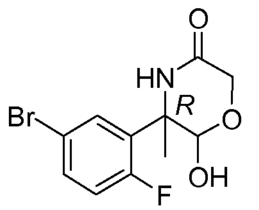

A solution of **intermediate 19** (10 g, 21.5 mmol) in THF (105 mL) was cooled to -78 °C under $N_2$ atmosphere. Then, diisobutylaluminium hydride (43 mL, 43 mmol) was slowly added. The reaction mixture was stirred for 2 hours allowing it to slowly warm up to room temperature. The reaction mixture was cooled down to 0 °C and quenched by slow addition of aqueous 1N HCl solution. The mixture was then extracted with EtOAc, the organic layers were separated, dried ($Na_2SO_4$), filtered and the solvents evaporated *in vacuo* to yield **intermediate 20** (6.6 g, quant. yield, mixture of diastereoisomers 80/20) which was used as such in the next reaction step.

Example A20

Preparation of **intermediate 21**.

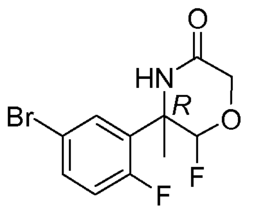

**Intermediate 20** (6.3 g, 20.7 mmol) was dissolved in DCM (84 mL) and the reaction was cooled down to 0 °C. Then DAST (3 mL, 24.9 mmol) was added dropwise. After 20 min at 0 °C the reaction mixture was quenched with aq. sat. $NaHCO_3$ solution and extracted with DCM. The combined organic layers were dried ($MgSO_4$), filtered, and the solvent evaporated *in vacuo.* The crude product was suspended from DIPE, filtered off and dried under vacuum at 60°C to yield **intermediate 21** (4.2 g, 66% yield, mixture of diastereoisomers 80/20).

Example A21

Preparation of **intermediate 22.**

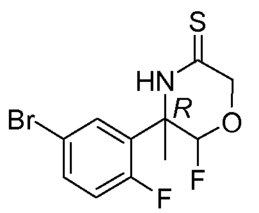

**Intermediate 22** was synthesized following the same approach described in the Example A9. Starting from **intermediate 21** (4.2 g, 13.7 mmol) **intermediate 22** was obtained (3 g, 68% yield, mixture of diastereoisomers 60/40).

Example A22

Preparation of **intermediate 23** and **24.**

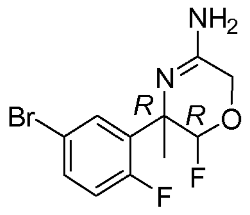

**intermediate 23**

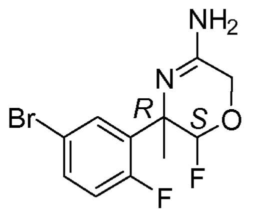

**intermediate 24**

**Intermediate 22** (6 g, 18.6 mmol) was dissolved in 7N ammonia in MeOH (300 mL) and the reaction mixture was stirred at 60 °C for 18 hours. The solvent was evaporated and additional 7N ammonia in MeOH was added (300 mL) and the mixture was stirred at 60 °C for an additional 18 hours. Then the solvent was evaporated and the crude product purified by column chromatography (silica gel; eluent: 7 M solution of ammonia in methanol/DCM 0/100 to 10/90). The desired fractions were collected and concentrated *in vacuo* to yield **intermediate 23** (3.7 g, 65% yield) and **intermediate 24** (0.6 g, 11% yield).

Example A23

Preparation of **intermediate 25.**

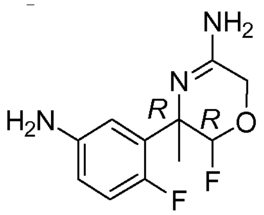

**Intermediate 23** (1.6 g, 5.24 mmol) was combined with $NaN_3$ (0.85 g, 13 mmol), CuI (1.25 g, 6.5 mmol) and $Na_2CO_3$ (1.1 g, 10.5 mmol) in DMSO (75 mL) and the reaction was degassed. After that, *N,N'*-dimethylethylenediamine (1 mL, 9.1 mmol) was added and the mixture was heated at 110 °C until completion of the reaction, about 4 hours. The reaction mixture was poured in DCM. Ammonium hydroxide (28% in water) was added and the organic layer was separated and washed three times with ammonium hydroxide. Then the organic layer was dried ($Mg_2SO_4$), filtered and concentrated *in vacuo.* The crude product was purified by column chromatography (silica gel; eluent: 7 M solution of ammonia in methanol/DCM 0/100 to 10/90). The desired fractions were collected and concentrated *in vacuo* to yield **intermediate 25** (0.3 g, 24% yield).

Example A24

Preparation of **intermediate 26.**

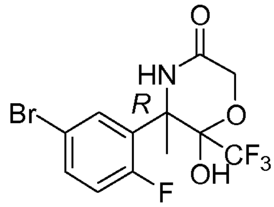

To a solution of **intermediate 19** (11.6 g, 38.5 mmol) in THF (117 mL) was added TBAT (2.08 g, 3.85 mmol). Then, (trifluoromethyl)trimethyl silane (12.5 mL, 84.6 mmol) was added dropwise, and the r.m. was stirred at room temperature for 20 minutes. The mixture was quenched with aqueous NaCl and extracted with EtOAc. The combined organic layers were dried ($MgSO_4$), filtered and concentrated *in vacuo* to yield **intermediate 26** (14 g, 98% yield) as a mixture of cis and trans isomers, which was used as such in the next step.

Example A25

Preparation of **intermediate 27**.

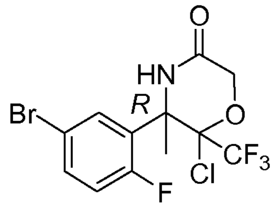

**Intermediate 26** (14 g, 37.6 mmol) was dissolved in DCM (600 mL) and cooled to 0 °C and then thionyl chloride (11.2 mL, 150 mmol) was added dropwise . The reaction mixture was stirred for 30 min at 0 °C and then pyridine (18.2 mL, 225.7 mmol) was added. After 30 minutes the reaction was hydrolyzed with an aqueous 1N HCl solution and then extracted with DCM. The organic layers were separated, dried ($MgSO_4$), filtered and evaporated *in vacuo.* The crude product was purified by flash column chromatography (silica gel; eluent: 7 M solution of ammonia in methanol/DCM 0/100 to 2/98). The desired fractions were collected and concentrated *in vacuo* to yield **intermediate 27** (6 g, 41% yield, mixture of diastereoisomers).

Example A26

Preparation of **intermediate 28.**

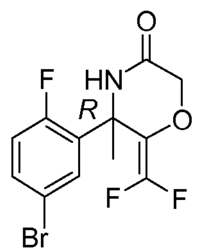

**Intermediate 27** (7 g, 17.9 mmol) and zinc copper couple (8.55 g, 66.3 mmol) were stirred in acetic acid (420 mL) at room temperature for 16 hours. The reaction mixture was filtered, washed with DCM and concentrated *in vacuo.* Ammonium hydroxide solution (28% in water) and DCM were added and the mixture was stirred at room temperature for one hour. The organic layer was separated and the aqueous layer was extracted with DCM. The combined organic layers were dried ($MgSO_4$), filtered and evaporated *in vacuo* to yield **intermediate 28** (6 g, 99% yield) as a white powder.

Example A27

Preparation of **intermediate 29**.

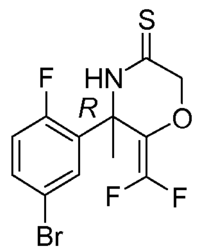

$P_2S_5$ (5.95 g, 26.8 mmol) was added to a solution of **intermediate 28** (6 g, 17.9 mmol) in THF (145 mL) at room temperature. The mixture was stirred at 70 °C for 90 minutes. Then the mixture was cooled to room temperature, filtered off and the organic solvent evaporated *in vacuo* to yield **intermediate 29** (5.9 g), which was used as such in the next step.

Example A28

Preparation of **intermediate 30.**

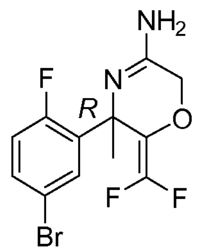

**Intermediate 30** was synthesized following the same approach described in the Example A22. Starting from **intermediate 29** (5.9 g, 16.8 mmol) **intermediate 30** was obtained (4.04 g, 72% yield).

Example A29

Preparation of **intermediate 31**.

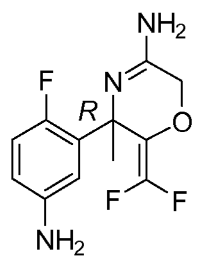

**Intermediate 31** was synthesized following the same approach described in the Example A23. Starting from **intermediate 30** (3.6 g, 10.7 mmol) **intermediate 31** was obtained (1.52 g, 52% yield).

Example A30

Preparation of **intermediate 32**.

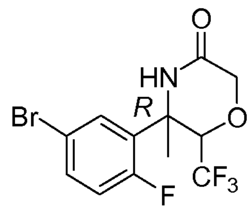

To a solution of **intermediate 27** (3 g, 7.68 mmol) in acetic acid (136 mL), zinc (1.26 g, 19.2 mmol) was added. The reaction mixture was then stirred at 80 °C for 3 hours, after that the reaction was filtered hot and concentrated *in vacuo.* The residue was dissolved in DCM and washed with ammonium hydroxide solution. The organic phase was separated, dried ($MgSO_4$) and the solvent concentrated *in vacuo.* The crude product purified by column chromatography (silica gel; eluent: 7 M solution of ammonia in methanol/DCM 0/100 to 3/97). The desired fractions were collected and concentrated *in vacuo* to yield **intermediate 32** (2.7 g, 99% yield).

Example A31

Preparation of **intermediate 33.**

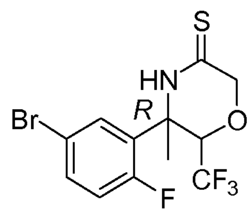

Lawesson's reagent (6.82 g, 16.85 mmol) was added to a solution of **intermediate 32** (6 g, 16.85 mmol) dissolved in THF (68 mL) at room temperature. The mixture was stirred at 60 °C for 4 hours. Then the mixture was cooled to room temperature, filtered off and the organic solvent evaporated *in vacuo.* The crude product was purified by flash column chromatography (silica gel; eluent: heptanes/DCM 100/0 to 50/50). The desired fractions were collected and evaporated *in vacuo* to yield **intermediate 33** (6 g, 96% yield) as a yellowish oil.

Example A32

Preparation of **intermediate 34** and **intermediate 35**

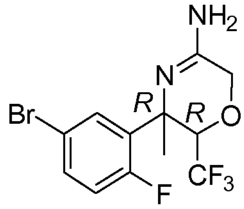

**intermediate 34**

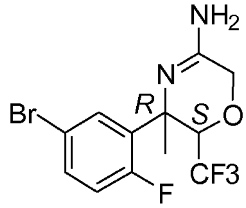

**intermediate 35**

**Intermediate 33** (6 g, 16.1 mmol) was dissolved in 7N ammonia in MeOH (97 mL) and the reaction mixture

was stirred at 80 °C for 24 hours. Then the solvent was evaporated and the crude product purified by column chromatography (silica gel; eluent: 7 M solution of ammonia in methanol/DCM 0/100 to 2/98). The desired fractions were collected and concentrated *in vacuo* to yield **intermediate 34** (3.4 g, 59% yield) and **intermediate 35** (0.75 g, 13% yield).

Example A33

Preparation of **intermediate 36.**

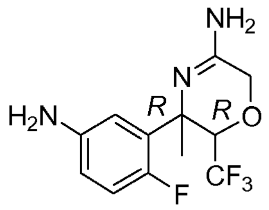

**Intermediate 34** (3.4 g, 9.6 mmol) was combined with $NaN_3$ (1.56 g, 24 mmol), CuI (2.28 g, 12 mmol) and $Na_2CO_3$ (2.03 g, 19.1 mmol) in DMSO (137 mL) and the reaction was degassed. After that, *N,N'*-dimethylethylenediamine (1.8 mL, 16.8 mmol) was added and the mixture was heated at 110 °C until completion of the reaction, about 1 hour. The reaction mixture was filtered off and the filter cake was washed with EtOAc. Water and EtOAc were added to the filtrate and the mixture was acidified by addition of HCl (1M in $H_2O$). The organic layer was then separated and the aqueous layer washed with EtOAc. Then the water layer was basified with an aq. ammonia solution and extracted again with EtOAc. The combined organic layers were dried, ($Na_2SO_4$) filtered and concentrated *in vacuo* to yield **intermediate 36** (2.5 g, 90% yield).
$\alpha_D$: -94.91° (0589 nm, c 0.393 w/v %, MeOH, 20 °C)

B. Preparation of the final compounds

Example B1

Preparation of **compound 1:** (5R,6S)-5-cyclopropyl-6-fluoro-5-{3-[(3-methoxypyridin-2-yl)amino]phenyl}-6-(trifluoromethyl)-5,6-dihydro-2H-1,4-oxazin-3-amine

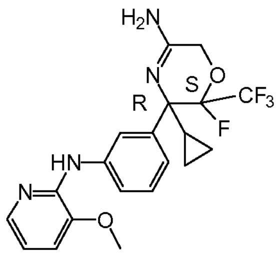

**Intermediate 12** (0.07 g, 0.221 mmol) was dissolved in isopropanol (5 mL) and 2-bromo-3-methoxypyridine (0.083 g, 0.441 mmol) and sulfuric acid (0.108 g, 1.1 mmol) were added. The mixture was stirred for 40 hours at 80 °C. The mixture was allowed to cool down to room temperature. DCM and sat. aq. $NaHCO_3$ solution were added. The organic layers was separated, dried ($MgSO_4$), filtered and the solvents evaporated *in vacuo.* The crude product was purified by flash column chromatography (silica gel; eluent: 7 M solution of ammonia in methanol/DCM 0/100 to 10/90). The desired fractions were collected and concentrated *in vacuo.* The residue was then dissolved in DIPE and converted to the HCl salt by addition of HCl in isopropanol. The resulting solid was filtered and dried *in vacuo* to yield **compound 1** (0.025 g, 25% yield) as hydrochloric salt.

Example B2

Preparation of **compound 2:** *(5R,6R)*-6-fluoro-5-{2-fluoro-5-[(3-methoxypyridin-2-yl)amino]phenyl}-5-methyl-5,6-dihydro-2H-1,4-oxazin-3-amine

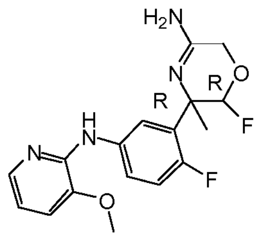

**Intermediate 25** (0.3 g, 1.24 mmol) was dissolved in isopropanol (15 mL) and 2-bromo-3-methoxypyridine (0.467 g, 2.49 mmol) and sulfuric acid (0.61 g, 6.22 mmol) were added. The mixture was stirred for 40 hours at 80 °C. The mixture was allowed to cool down to room temperature. DCM and sat. aq. $NaHCO_3$ solution were added. The organic layers was separated, dried ($MgSO_4$), filtered and the solvents evaporated *in vacuo.* The crude product was purified by preparative HPLC on (RP Vydac Denali® C18 - 10μm, 200 g, 5 cm). Mobile phase (0.25% NH4HCO3 solution in water, CH3CN) to yield **compound 2** (0.044 g, 10% yield).

Example B3

Preparation of **compound 3:** *(5R)*-6-(difluoromethylidene)-5-{2-fluoro-5-[(3-methoxy-pyridin-2-yl)amino]phenyl}-5-methyl-5,6-dihydro-2H-1,4-oxazin-3-amine

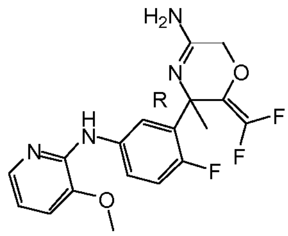

**Intermediate 31** (0.35 g, 1.29 mmol) was dissolved in isopropanol (15 mL) and 2-bromo-3-methoxypyridine (0.485 g, 2.58 mmol) and sulfuric acid (0.34 mL, 6.45 mmol) were added. The mixture was stirred for 72 hours at 80 °C. The mixture was allowed to cool down to room temperature. DCM and sat. aq. $NaHCO_3$ solution were added. The organic layers was separated, dried ($MgSO_4$), filtered and the solvents evaporated *in vacuo.* The crude product was purified by flash column chromatography (silica gel; eluent: 7 M solution of ammonia in methanol/DCM 0/100 to 2/98). The desired fractions were collected and concentrated *in vacuo.* The residue was suspended from DIPE/heptanes, filtered and dried under high vacuum to yield **compound 3** (0.279 g, 57% yield) as a white powder.

Example B4

Preparation of **compound 4:** (5R,6R*)-6-(Difluoromethyl)-5-12-fluoro-5-[(3-methoxy-pyridin-2-yl)amino]phenyl}-5-methyl-5,6-dihydro-2H-1,4-oxazin-3-amine

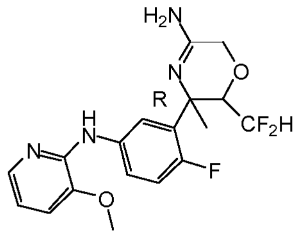

**Compound 3** (0.228 g, 0.603 mmol) was dissolved in EtOAc (4 mL) and palladium on carbon (10%) (0.064 g, 0.06 mmol) and thiophene (0.4% solution in THF, 0.8 mL, 0.041 mmol) were added. The mixture was hydrogenated at rt and atmospheric pressure for 16 hours. The catalyst was filtered off and the solvents evaporated *in vacuo.* The crude product was purified by flash column chromatography (silica gel; eluent: 7 M solution of ammonia in methanol/DCM 0/100 to 2/98). The desired fractions were collected and concentrated *in vacuo.* The residue was suspended from DIPE/heptanes, filtered and dried under high vacuum to yield **compound 4** (0.074 g, 32% yield).

Example B5

Preparation of **compound 5:** *(5R, 6R)*-6-fluoro-5-[2-fluoro-5-(pyrimidin-2-ylamino) phenyl]-5-methyl-5,6-dihydro-2H-1,4-oxazin-3-amine

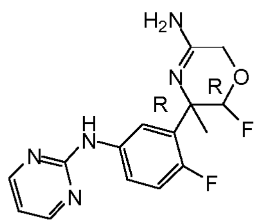

**Intermediate 25** (0.048 g, 0.199 mmol) was dissolved in 1,4-dioxane (2 mL) and 2-bromopyrimidine (0.032 g, 0.199 mmol) and 4M HCl solution in dioxane (0.1 mL, 0.4 mmol) were added. The mixture was stirred for 16 hours at 100 °C. The mixture was allowed to cool down to room temperature. DCM and sat. aq. $NaHCO_3$ solution were added. The organic layers was separated, dried ($MgSO_4$), filtered and the solvents evaporated *in vacuo.* The crude product was purified by flash column chromatography (silica gel; eluent: 7 M solution of ammonia in methanol/DCM 0/100 to 5/95). The desired fractions were collected and concentrated *in vacuo* to yield **compound 5** (0.013 g, 20% yield).

Example B6

Preparation of **compound 6:** *(5R, 6R)*-5-{2-fluoro-5-[(3-methoxypyridin-2-yl)amino] phenyl}-5-methyl-6-(trifluoromethyl)-5,6-dihydro-2H-1,4-oxazin-3-amine

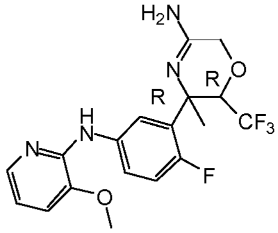

**Intermediate 36** (0.1 g, 0.343 mmol) was dissolved in isopropanol (4 mL) and 2-bromo-3-methoxypyridine (0.129 g, 0.687 mmol) and sulfuric acid (0.09 mL, 1.72 mmol) were added. The mixture was stirred for 40 hours at 80 °C. The mixture was allowed to cool down to room temperature. DCM and sat. aq. $NaHCO_3$ solution were added. The organic layers was separated, dried ($MgSO_4$), filtered and the solvents evaporated *in vacuo.* The crude product was

purified by flash column chromatography (silica gel; eluent: 7 M solution of ammonia in methanol/DCM 0/100 to 5/95) to yield **compound 6** (0.013 g, 20% yield).

Example B7

Preparation of **compound 7:** *(5R,6R)*-5-[2-fluoro-5-(pyrimidin-2-ylamino)phenyl]-5-methyl-6-(trifluoromethyl)-5,6-dihydro-2H-1,4-oxazin-3-amine

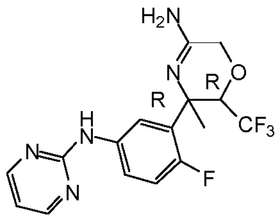

**Intermediate 36** (0.1 g, 0.343 mmol) was dissolved in 1,4-dioxane (3.4 mL) and 2-bromopyrimidine (0.055 g, 0.343 mmol) and 4M HCl solution in dioxane (0.17 mL, 0.69 mmol) were added. The mixture was stirred for 16 hours at 100 °C. The mixture was allowed to cool down to room temperature. DCM and sat. aq. $NaHCO_3$ solution were added. The organic layers was separated, dried ($MgSO_4$), filtered and the solvents evaporated *in vacuo.* The crude product was purified by flash column chromatography (silica gel; eluent: 7 M solution of ammonia in methanol/DCM 0/100 to 5/95). The desired fractions were collected and concentrated. This crude was The crude product was purified by preparative HPLC on (Chiralpal Diacel AS 20x250mm). Mobile phase ($CO_2$, MeOH with 0.2% iPrNH2) to yield **compound 7** (0.036 g, 28% yield).

Example B8

Preparation of **compound 8:** *(5R,6R)*-6-fluoro-5-{2-fluoro-5-[(3-methoxypyrazin-2-yl)amino]phenyl}-5-methyl-5,6-dihydro-2*H*-1,4-oxazin-3-amine

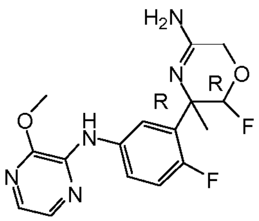

**Intermediate 25** (0.15 g, 0.622 mmol) was dissolved in 1,4-dioxane (6 mL). 2-Iodo-3-methoxypyrazine (0.12 g, 0.508 mmol), cesium carbonate (0.405 g, 1.244 mmol, 1,1'-bis(diphenylphosphino)ferrocene (0.052 g, 0.093 mmol) and tris(dibenzylideneacetone) dipalladium(0) (0.028 g, 0.031 mmol) were added. The reaction tube was sealed and the mixture was stirred at 160 °C for 1 hour under microwave irradiation. After cooling, the reaction mixture was diluted with DCM and filtered over dicalite. The filtrate was concentrated *in vacuo.* The crude product was purified by flash column chromatography (silica gel; eluent: 7 M solution of ammonia in methanol/DCM 0/100 to 5/95). The desired fractions were collected and concentrated. This crude was further purified by preparative HPLC on (Chiralpal Diacel AS 20x250mm). Mobile phase ($CO_2$, MeOH with 0.2% iPrNH2) to yield **compound 8** (0.010 g, 5% yield).

Example B9

Preparation of **compound 9:**

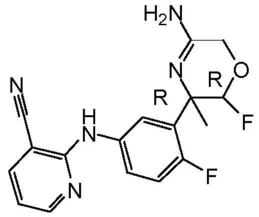

**Intermediate 25** (0.30 g, 1.244 mmol) and 2-bromonicotinonitrile (455 mg, 2.487 mmol) were dissolved in 1,4-dioxane (12.5 mL), then a 4M HCl solution in dioxane (0.933 mL, 3.731 mmol) was added dropwise. The reaction mixture was stirred for 16 hours at 120°C. A sat. aq. $NaHCO_3$ solution was then added, followed by DCM. The phases were separated and the organic layer was concentrated under reduced pressure. The resulting oil was purified by flash column chromatography (silica gel; elueng : 7M solution of ammonia in methanol/DCM 0/100 to 5/95). The desired fractions were collected and further purified by preparative SFC on (Chiralcel Diacel OD 20 x 250 mm). Mobile phase ($CO_2$, iPrOH with 0.2% $iPrNH_2$), to yield **compound 9** (0.006 g, 1%)

Example B10

Preparation of **compound 10:**

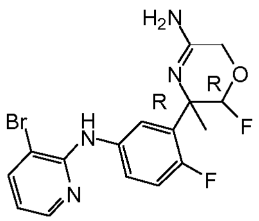

**Intermediate 25** (0.482 g, 2 mmol) was dissolved in iPrOH (24 mL), then 2,3-dibromopyridine (0.948 g, 4 mmol) and sulfuric acid (0.533 mL, 10 mmol) were added. The reaction mixture was stirred for 4 days at 80°C. The reaction was allowed to cool down, then DCM and a sat. aq. $NaHCO_3$ solution were then added. The phases were separated and the organic layer was dried and concentrated under reduced pressure. The resulting oil was purified by flash column chromatography (silica gel; elueng : 7M solution of ammonia in methanol/DCM 0/100 to 10/90). The desired fractions were collected and further purified by preparative SFC (Chiralpak Daicel AS 20 microhm 500 gr). Mobile phase ($CO_2$, iPrOH with 0.2% $iPrNH_2$), to yield **compound 10** (0.135 g, 17%)

Example B11

Preparation of **compound 11**

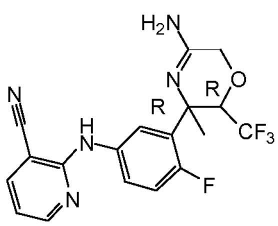

**Intermediate 36** (0.358 g, 1.229 mmol) was dissolved in 1,4-dioxane (14.7 mL). 2-Bromo-3-cyanopyridine (0.191 g, 1.045 mmol), cesium carbonate (0.800 g, 2.458 mmol), 1,1'-bis(diphenylphosphino)ferrocene (0.104 g, 0.184 mmol) were added and the mixture stirred under argon atmosphere for few minutes. Tris(dibenzylidene-acetone)dipalladium(O) (0.056 g, 0.062 mmol) was then added. The reaction tube was sealed and the mixture was stirred at 160 °C for 1 hour under microwave irradiation. After cooling, the reaction mixture was diluted with DCM and filtered over dicalite. The filtrate was concentrated *in vacuo.* The crude product was purified by flash column chromatography (silica gel;

eluent: 7 M solution of ammonia in methanol/DCM 0/100 to 5/95). The desired fractions were collected and concentrated. This crude was further purified by preparative SFC on (Chiralpal Diacel AS 20x250mm). Mobile phase ($CO_2$, MeOH with 0.2% $iPrNH_2$) to yield **compound 11** (0.052 g, 11% yield).

Compounds 1 to 11 in tables 1-5 list the compounds that were prepared by analogy to one of the above Examples. In case no salt form is indicated, the compound was obtained as a free base. 'Ex. No.' refers to the Example number according to which protocol the compound was synthesized. 'Co. No.' means compound number.

**Table 1:**

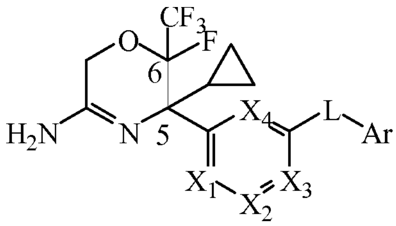

| Co. No. | Ex. No. | $X_1$, $X_2$, $X_3$, $X_4$ | ---L-Ar | stereochemistry |
|---|---|---|---|---|
| **1** | B1 | $X_1=X_2=X_3=X_4=CH$ | 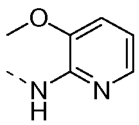 | $C_5$(R);$C_6$(S) Single diastereoisomer Pure enantiomer |

**Table 2:**

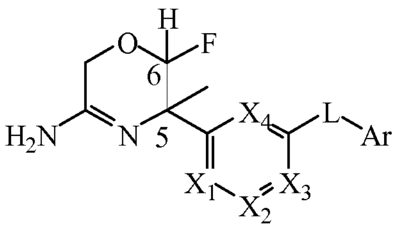

| Co. No. | Ex. No. | $X_1$, $X_2$, $X_3$, $X_4$ | ---L-Ar | stereochemistry |
|---|---|---|---|---|
| **2** | B2 | $X_1=CF$<br>$X_2=X_3=X_4=CH$ | 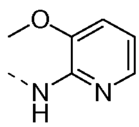 | $C_5$(R);$C_6$(R)<br>Single diastereoisomer<br>Pure enantiomer |
| **5** | B5 | $X_1=CF$<br>$X_2=X_3=X_4=CH$ | 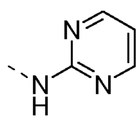 | $C_5$(R);$C_6$(R)<br>Single diastereoisomer<br>Pure enantiomer |
| **9** | B9 | $X_1=CF$<br>$X_2=X_3=X_4=CH$ | 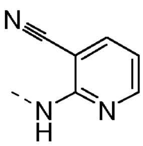 | $C_5$(R);$C_6$(R)<br>Single diastereoisomer<br>Pure enantiomer |
| **10** | B10 | $X_1=CF$<br>$X_2=X_3=X_4=CH$ | 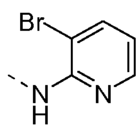 | $C_5$(R);$C_6$(R)<br>Single diastereoisomer<br>Pure enantiomer |

**Table 3:**

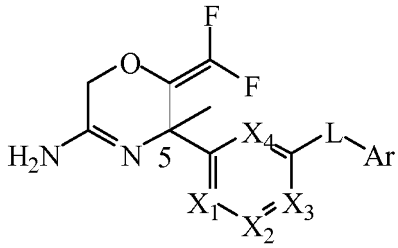

| Co. No. | Ex. No. | $X_1$, $X_2$, $X_3$, $X_4$ | ---L-Ar | stereochemistry |
|---|---|---|---|---|
| **3** | B3 | $X_1$=CF<br>$X_2$=$X_3$=$X_4$=CH | 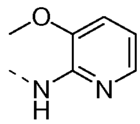 | $C_5$(R)<br>Pure enantiomer |

**Table 4:**

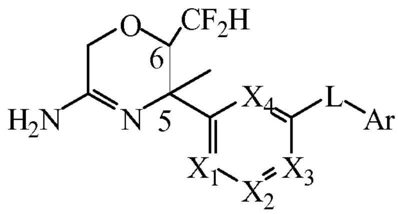

| Co. No. | Ex. No. | $X_1$, $X_2$, $X_3$, $X_4$ | ---L-Ar | stereochemistry |
|---|---|---|---|---|
| **4** | B4 | $X_1$=CF<br>$X_2$=$X_3$=$X_4$=CH | 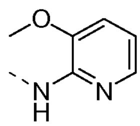 | $C_5$(R);$C_6$(R*)<br>Single diastereoisomer<br>Pure enantiomer |

**Table 5:**

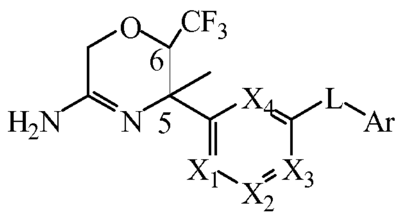

| Co. No. | Ex. No. | $X_1$, $X_2$, $X_3$, $X_4$ | ---L-Ar | stereochemistry |
|---|---|---|---|---|
| **6** | B6 | $X_1$=CF<br>$X_2$= $X_3$= $X_4$=CH | 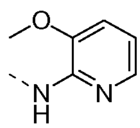 | $C_5$(R);$C_6$(R)<br>Single diastereoisomer<br>Pure enantiomer |
| **7** | B7 | $X_1$=CF<br>$X_2$=$X_3$=$X_4$=CH | 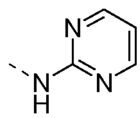 | $C_5$(R);$C_6$(R)<br>Single diastereoisomer<br>Pure enantiomer |

(continued)

| Co. No. | Ex. No. | $X_1$, $X_2$, $X_3$, $X_4$ | ---L-Ar | stereochemistry |
|---|---|---|---|---|
| **11** | B11 | $X_1$=CF<br>$X_2$=$X_3$=$X_4$=CH | 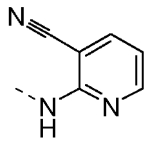 | $C_5$(R);$C_6$(R)<br>Single diastereoisomer<br><br>Pure enantiomer |

C. Analytical Part

**LCMS**

For (LC)MS-characterization of the compounds of the present invention, the following methods were used.

*General procedure:*

The LC measurement was performed using an Acquity UPLC (Waters) system comprising a binary pump, a sample organizer, a column heater (set at 55 °C), a diode-array detector (DAD) and a column as specified in the respective methods below. Flow from the column was split to a MS spectrometer. The MS detector was configured with an electrospray ionization source. Mass spectra were acquired by scanning from 100 to 1000 in 0.18 seconds using a dwell time of 0.02 seconds. The capillary needle voltage was 3.5 kV and the source temperature was maintained at 140 °C. Nitrogen was used as the nebulizer gas. Data acquisition was performed with a Waters-Micromass MassLynx-Openlynx data system.

*Method 1*:Reversed phase UPLC (Ultra Performance Liquid Chromatography) was carried out on a bridged ethylsiloxane/silica hybrid (BEH) C18 column (1.7 μm, 2.1 x 50 mm; Waters Acquity) with a flow rate of 0.8 ml/min. Two mobile phases (mobile phase A: 10mM ammonium acetate in $H_2O$/acetonitrile 95/5; mobile phase B: acetonitrile) were used to run a gradient condition from 95 % A and 5 % B to 5 % A and 95 % B in 1.3 minutes and hold for 0.2 minutes. An injection volume of 0.5 μl was used.

Cone voltage was 10 V for positive ionization mode and 20 V for negative ionization mode.

*Method 2:*

Reversed phase UPLC (Ultra Performance Liquid Chromatography) was carried out on a bridged ethylsiloxane/silica hybrid (BEH) C18 column (1.7 μm, 2.1 x 50 mm; Waters Acquity) with a flow rate of 0.8 ml/min. Two mobile phases (10 mM ammonium acetate in $H_2O$/acetonitrile 95/5; mobile phase B: acetonitrile) were used to run a gradient condition from 95 % A and 5 % B to 5 % A and 95 % B in 1.3 minutes and hold for 0.3 minutes. An injection volume of 0.5 μl was used.

Cone voltage was 10 V for positive ionization mode and 20 V for negative ionization mode.

*Method 3:*

Reversed phase UPLC (Ultra Performance Liquid Chromatography) was carried out on a bridged ethylsiloxane/silica hybrid (BEH) C18 column (1.7 μm, 2.1 x 50 mm; Waters Acquity) with a flow rate of 0.8 ml/min. Two mobile phases (10 mM ammonium acetate in $H_2O$/acetonitrile 95/5; mobile phase B: acetonitrile) were used to run a gradient condition from 95 % A and 5 % B to 5 % A and 95 % B in 1.3 minutes and hold for 0.3 minutes. An injection volume of 0.5 μl was used.

Cone voltage was 10 V for positive ionization mode and 20 V for negative ionization mode.

**Melting Points**

Values are either peak values or melt ranges, and are obtained with experimental uncertainties that are commonly associated with this analytical method.

DSC823e (indicated by DSC in Table 6)

For a number of compounds, melting points were determined with a DSC823e (Mettler-Toledo). Melting points

were measured with a temperature gradient of 30° C/minute. Maximum temperature was 400°C.

**Table 6: Analytical data -** $R_t$ means retention time (in minutes), $[M+H]^+$ means the protonated mass of the compound, method refers to the method used for (LC)MS.

| Co.Nr. | $R_t$ | $[M+H]^+$ | Method | Melting Point |
|---|---|---|---|---|
| **1** | 1.10 | 425 | 2 | n.d. |
| **2** | 0.81 | 349 | 2 | n.d. |
| **3** | 0.90 | 379 | 2 | b.r. |
| **4** | 0.78 | 381 | 2 | 136.93°C |
| **5** | 0.67 | 320 | 1 | n.d. |
| **6** | 0.94 | 399 | 2 | n.d. |
| **7** | 0.8 | 370 | 2 | n.d. |
| **8** | 0.82 | 350 | 3 | n.d. |
| **9** | 0.81 | 344 | 1 | n.d. |
| **10** | 0.90 | 398 | 2 | n.d. |
| **11** | 0.87 | 394 | 3 | n.d. |
| n.d. means not determined, b.r. means broad range | | | | |

**Optical Rotations**

Optical rotations were measured on a Perkin-Elmer 341 polarimeter with a sodium lamp and reported as follows: $[\alpha]_\lambda^{t°C}$ (c g/100 ml, solvent).

**Table 7: Analytical data - Optical rotation values for enantiomerically pure compounds**

| Co. Nr. | $\alpha_D$(°) | Wavelength (nm) | Concentration w/v % | Solvent | Temp. (° C) |
|---|---|---|---|---|---|
| **3** | -163.16 | 589 | 0.456 | DMF | 20 |
| **4** | +196.03 | 365 | 0.1765 | DMF | 20 |
| **6** | +9.12 | 365 | 0.57 | DMF | 20 |

**NMR**

For a number of compounds, $^1H$ NMR spectra were recorded on a Bruker DPX-360, on a Bruker DPX-400 or on a Bruker Avance 600 spectrometer with standard pulse sequences, operating at 360 MHz, 400 MHz and 600 MHz respectively, using CHLOROFORM-*d* (deuterated chloroform, $CDCl_3$) or DMSO-$d_6$ (deuterated DMSO, dimethyl-d6 sulfoxide) as solvents. Chemical shifts (δ) are reported in parts per million (ppm) relative to tetramethylsilane (TMS), which was used as internal standard.

**Table 8:**

| Co. Nr. | NMR result |
|---|---|
| **4** | $^1H$ NMR (360 MHz, DMSO-$d_6$) d ppm 1.52 (s, 3 H) 3.87 (s, 3 H) 3.90 - 4.04 (m, 1 H) 4.06 - 4.21 (m, 2 H) 5.43 - 5.75 (m, 1 H) 5.77 (br. s., 2 H) 6.73 (dd, *J*=7.7, 5.1 Hz, 1 H) 6.99 (dd, *J*=11.7, 8.8 Hz, 1 H) 7.19 (d, *J*=7.0 Hz, 1 H) 7.71 (d, *J*=4.0 Hz, 1 H) 7.85 (ddd, *J*=8.2, 3.7, 3.5 Hz, 1 H) 7.91 (dd, *J*=7.0, 2.6 Hz, 1 H) 8.04 (s, 1H) |
| **5** | $^1H$ NMR (360 MHz, $CDCl_3$-*d*): δ 1.62 (t, *J*=1.8 Hz, 3 H) 3.24-3.85 (m, 2 H) 3.95 - 4.28 (m, 2 H) 5.93 - 6.14 (m, 1 H) 6.68 (t, *J*=4.76 Hz, 1 H) 7.02 (dd, *J*=11.53, 8.96 Hz, 1 H) 7.32 (dd, *J*=6.95, 2.93 Hz, 1 H) 7.63 - 7.74 (m, 2 H) 8.37 (d, *J*=4.76 Hz, 2 H) |

(continued)

| Co. Nr. | NMR result |
|---|---|
| **6** | $^1$H NMR (360 MHz, $CDCl_3$-*d*): δ 1.67 (s, 3 H) 3.90 (s, 3 H) 4.22 (s, 2 H) 4.65 (q, *J*=8.40 Hz, 1 H) 6.68 (dd, *J*=8.05, 5.12 Hz, 1 H) 6.91 - 7.04 (m, 2 H) 7.08 (s, 1 H) 7.65 (dd, *J*=6.59, 2.93 Hz, 1 H) 7.80 (dd, *J*=5.12, 1.10 Hz, 1 H) 8.07 - 8.17 (m, 1 H) |
| **7** | $^1$H NMR (360 MHz,, $CDCl_3$-*d*): δ 1.68 (s, 3 H) 4.22 (s, 4 H) 4.63 (q, *J*=8.05 Hz, 1 H) 6.70 (t, *J*=4.76 Hz, 1 H) 7.01 (dd, *J*=11.34, 8.78 Hz, 1 H) 7.22 (s, 1 H) 7.70 (dd, *J*=6.59, 2.93 Hz, 1 H) 7.89 (dt, *J*=8.78, 3.66 Hz, 1 H) 8.40 (d, *J*=4.76 Hz, 2 H) |
| **8** | $^1$H NMR (360 MHz, , $CDCl_3$-*d*): δ 1.65 (t, *J*=1.83 Hz, 3 H) 3.97-4.30 (m, 5 H) 5.95 - 6.17 (m, 1 H) 6.95 - 7.10 (m, 2 H) 7.39 (dd, *J*=6.95, 2.93 Hz, 1 H) 7.46 (d, *J*=2.93 Hz, 1 H) 7.64 (d, *J*=3.29 Hz, 1 H) 7.86 (ddd, *J*=8.78, 4.03, 2.93 Hz, 1 H) |
| **9** | $^1$H NMR (360 MHz, $CDCl_3$-*d*): d 1.21 (d, *J*=6.22 Hz, 3 H) 3.98-4.30 (m, 2 H) 5.91 - 6.15 (m, 1 H) 6.78 (dd, *J*=7.68, 4.76 Hz, 1 H) 6.95 - 7.11 (m, 2 H) 7.31 (dd, *J*=6.77, 2.74 Hz, 1 H) 7.64-7.72 (m, 1 H) 7.75 - 7.83 (m, 1 H) 8.33 (dd, *J*=5.12, 1.83 Hz, 1 H) |
| **10** | $^1$H NMR (360 MHz, $CDCl_3$-*d*): d 1.66 (t, *J*=1.65 Hz, 3 H) 3.97-4.30 (m, 2 H) 5.94 - 6.16 (m, 1 H) 6.62 (dd, *J*=7.68, 4.76 Hz, 1 H) 6.93 (s, 1 H) 7.04 (dd, *J*=11.34, 8.78 Hz, 1 H) 7.32 (dd, *J*=6.95, 2.93 Hz, 1 H) 7.65 - 7.78 (m, 2 H) 8.10 (dd, *J*=4.76, 1.46 Hz, 1 H) |
| **11** | $^1$H NMR (360 MHz, $CDCl_3$-*d*): d ppm 1.67 (s, 3 H) 4.14 - 4.34 (m, 4 H) 4.65 (d, *J*=8.42 Hz, 1 H) 6.78 (dd, *J*=7.50, 4.94 Hz, 1 H) 6.98 - 7.08 (m, 2 H) 7.71 (dd, *J*=6.59, 2.93 Hz, 1 H) 7.78 (dd, J=7.68, 1.83 Hz, 1 H) 7.94 (ddd, *J*=8.78, 4.03, 2.93 Hz, 1 H) 8.37 (dd, *J*=5.12, 1.83 Hz, 1 H) |

D. Pharmacological examples

The compounds provided in the present invention are inhibitors of the beta-site APP-cleaving enzyme 1 (BACE1). Inhibition of BACE1, an aspartic protease, is believed to be relevant for treatment of Alzheimer's Disease (AD). The production and accumulation of beta-amyloid peptides (Abeta) from the beta-amyloid precursor protein (APP) is believed to play a key role in the onset and progression of AD. Abeta is produced from the amyloid precursor protein (APP) by sequential cleavage at the N- and C-termini of the Abeta domain by beta-secretase and gamma-secretase, respectively.

Compounds of Formula (I) are expected to have their effect substantially at BACE1 by virtue of their ability to inhibit the enzymatic activity. The behaviour of such inhibitors tested using a biochemical Fluorescence Resonance Energy Transfer (FRET) based assay and a cellular αLisa assay in SKNBE2 cells described below and which are suitable for the identification of such compounds, and more particularly the compounds according to Formula (I), are shown in Table 9 and Table 10.

*Biochemical FRET based essay*

This assay is a Fluorescence Resonance Energy Transfer Assay (FRET) based assay. The substrate for this assay is an APP derived 13 amino acids peptide that contains the 'Swedish' Lys-Met/Asn-Leu mutation of the amyloid precursor protein (APP) beta-secretase cleavage site. This substrate also contains two fluorophores: (7-methoxycoumarin-4-yl) acetic acid (Mca) is a fluorescent donor with excitation wavelength at 320 nm and emission at 405 nm and 2,4-Dinitrophenyl (Dnp) is a proprietary quencher acceptor. The distance between those two groups has been selected so that upon light excitation, the donor fluorescence energy is significantly quenched by the acceptor, through resonance energy transfer. Upon cleavage by BACE1, the fluorophore Mca is separated from the quenching group Dnp, restoring the full fluorescence yield of the donor. The increase in fluorescence is linearly related to the rate of proteolysis.

Briefly in a 384-well format recombinant BACE1 protein in a final concentration of 1 μg/ml is incubated for 120 minutes at room temperature with 10 μm substrate in incubation buffer (40 mM Citrate buffer pH 5.0, 0.04 % PEG, 4 % DMSO) in the absence or presence of compound. Next the amount of proteolysis is directly measured by fluorescence measurement at T=0 and T=120 (excitation at 320 nm and emission at 405 nm). Results are expressed in RFU (Relative Fluorescence Units), as difference between T120 and T0.

A best-fit curve is fitted by a minimum sum of squares method to the plot of %Controlmin versus compound concentration. From this an $IC_{50}$ value (inhibitory concentration causing 50% inhibition of activity) can be obtained.
LC = Median of the low control values
= Low control: Reaction without enzyme

HC = Median of the High control values
= High Control: Reaction with enzyme

$$\%Effect = 100-[(sample-LC) / (HC-LC) *100]$$

$$\%Control = (sample /HC)*100$$

$$\%Controlmin = (sample-LC) / (HC-LC) *100$$

The following exemplified compounds were tested essentially as described above and exhibited the following the activity:

**Table 9:**

| Co. Nr. | Biochemical FRET based assay $PIC_{50}$ |
|---|---|
| **1** | 7.58 |
| **2** | 6.79 |
| **3** | 6.12 |
| **4** | 7.23 |
| **5** | 5.71 |
| **6** | 6.86 |
| **7** | 5.43 |
| **8** | 6.51 |
| **9** | 6.65 |
| **10** | n.t. |
| **11** | n.t. |

*Cellular* αLisa *assay in SKNBE2 cells*

In two αLisa assays the levels of Abeta total and Abeta 1-42 produced and secreted into the medium of human neuroblastoma SKNBE2 cells are quantified. The assay is based on the human neuroblastoma SKNBE2 expressing the wild type Amyloid Precursor Protein (hAPP695). The compounds are diluted and added to these cells, incubated for 18 hours and then measurements of Abeta 1-42 and Abeta total are taken. Abeta total and Abeta 1-42 are measured by sandwich αLisa. αLisa is a sandwich assay using biotinylated antibody AbN/25 attached to streptavidin coated beads and antibody Ab4G8 or cAb42/26 conjugated acceptor beads for the detection of Abeta total and Abeta 1-42 respectively. In the presence of Abeta total or Abeta 1-42, the beads come into close proximity. The excitation of the donor beads provokes the release of singlet oxygen molecules that trigger a cascade of energy transfer in the acceptor beads, resulting in light emission. Light emission is measured after 1 hour incubation (excitation at 650 nm and emission at 615 nm).

A best-fit curve is fitted by a minimum sum of squares method to the plot of %Controlmin versus compound concentration. From this an $IC_{50}$ value (inhibitory concentration causing 50 % inhibition of activity) can be obtained.
LC = Median of the low control values
= Low control: cells preincubated without compound, without biotinylated Ab in the αLisa
HC = Median of the High control values
= High Control: cells preincubated without compound

$$\%Effect = 100-[(sample-LC) / (HC-LC) *100]$$

$$\%Control = (sample /HC)*100$$

$$\%Controlmin = (sample-LC) / (HC-LC) *100$$

The following exemplified compounds were tested essentially as described above and exhibited the following the activity:

**Table 10:**

| Co. Nr. | Cellular αLisa assay in SKNBE2 cells Abeta 42 $pIC_{50}$ | Cellular αLisa assay in SKNBE2 cells Abetatotal $pIC_{50}$ |
|---|---|---|
| **1** | 7.22 | 7.27 |
| **2** | 7.62 | 7.71 |
| **3** | 6.98 | 7.05 |
| **4** | 8.04 | 7.98 |
| **5** | 5.9 | 5.96 |
| **6** | 7.67 | 7.68 |
| **7** | 6.12 | 6.08 |
| **8** | 6.89 | 6.86 |
| **9** | 6.89 | 7.06 |
| **10** | n.t. | n.t. |
| **11** | n.t. | n.t. |
| n.t. means not tested | | |

**Claims**

**1.** A compound of Formula (I)

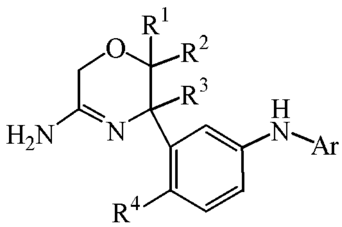

or a tautomer or a stereoisomeric form thereof, wherein

$R^1$ is fluoro, fluoromethyl, difluoromethyl or trifluoromethyl;
$R^2$ is hydrogen or trifluoromethyl; or
$R^1$ and $R^2$ form a divalent radical $=CF_2$;
$R^3$ is hydrogen, $C_{1-3}$alkyl, cyclopropyl, mono- or polyhalo-$C_{1-3}$alkyl;
$R^4$ is hydrogen or fluoro;
Ar is homoaryl or heteroaryl;
wherein homoaryl is phenyl or phenyl substituted with one, two or three substituents selected from the group consisting of halo, cyano, $C_{1-3}$alkyl, $C_{1-3}$alkyloxy, mono- and polyhalo-$C_{1-3}$alkyl, mono-and polyhalo-$C_{1-3}$alkyloxy;
heteroaryl is selected from the group consisting of pyridyl, pyrimidyl, pyrazyl, pyridazyl, furanyl, thienyl, pyrrolyl,

pyrazolyl, imidazolyl, triazolyl, tetrazolyl, thiazolyl, isothiazolyl, thiadiazolyl, oxazolyl, isoxazolyl, and oxadiazolyl, each optionally substituted with one, two or three substituents selected from the group consisting of halo, cyano, $C_{1-3}$alkyl, $C_{2-3}$alkynyl, $C_{1-3}$alkyloxy, mono- and polyhalo-$C_{1-3}$alkyl, mono- and polyhalo-$C_{1-3}$alkyloxy, and $C_{1-3}$alkyloxy$C_{1-3}$alkyloxy;

or an addition salt thereof.

**2.** The compound of claim 1 wherein
$R^1$ is fluoro, difluoromethyl or trifluoromethyl;
$R^2$ is hydrogen or trifluoromethyl; or
$R^1$ and $R^2$ form a divalent radical $=CF_2$;
$R^3$ is $C_{1-3}$alkyl, cyclopropyl, mono- or polyhalo-$C_{1-3}$alkyl;
$R^4$ is hydrogen or fluoro;
Ar is heteroaryl;
wherein heteroaryl is pyridyl or pyrimidyl, each optionally substituted with one, two or three substituents selected from the group consisting of halo, cyano, $C_{1-3}$alkyl, $C_{2-3}$alkynyl, $C_{1-3}$alkyloxy, mono- and polyhalo-$C_{1-3}$alkyl, mono- and polyhalo-$C_{1-3}$alkyloxy, and $C_{1-3}$alkyloxy$C_{1-3}$alkyloxy;
or an addition salt thereof.

**3.** The compound of claim 1 wherein
$R^1$ is fluoro, difluoromethyl or trifluoromethyl, and $R^2$ is hydrogen; or
$R^1$ is fluoro and $R^2$ is trifluoromethyl; or
$R^1$ and $R^2$ form a divalent radical $=CF_2$;
$R^3$ is methyl or cyclopropyl;
$R^4$ is hydrogen or fluoro;
Ar is heteroaryl;
wherein heteroaryl is pyridyl substituted with methoxy, or pyrimidyl;
or an addition salt thereof.

**4.** The compound of claim 1 wherein the carbon atom substituted with $R^3$ has the R configuration.

**5.** The compound of claim 1 selected from

(5*R*,6*S*)-5-cyclopropyl-6-fluoro-5-{3-[(3-methoxypyridin-2-yl)amino]phenyl}-6-(trifluoromethyl)-5,6-dihydro-2H-14-oxazin-3-amine;
(5*R*,6*R*)-6-fluoro-5-{2-fluoro-5-[(3-methoxypyridin-2-yl)amino]phenyl}-5-methyl-5,6-dihydro-2H-1,4-oxazin-3-amine,
(5*R*)-6-(difluoromethylidene)-5-{2-fluoro-5-[(3-methoxypyridin-2-yl)amino]phenyl 5-methyl-5,6-dihydro-2H-1,4-oxazin-3-amine;
(5*R*,6*R**)-6-(Difluoromethyl)-5-{2-fluoro-5-[(3-methoxypyridin-2-yl)amino]phenyl}-5-methyl-5,6-dihydro-2H-1,4-oxazin-3-amine;
(5*R*,6*R*)-6-fluoro-5-[2-fluoro-5-(pyrimidin-2-ylamino)phenyl]-5-methyl-5,6-dihydro-2H-1,4-oxazin-3-amine;
(5*R*,6*R*)-5-{2-fluoro-5-[(3-methoxypyridin-2-yl)amino]phenyl}-5-methyl-6-(trifluoromethyl)-5,6-dihydro-2H-1,4-oxazin-3-amine;
(5*R*,6*R*)-5-[2-fluoro-5-(pyrimidin-2-ylamino)phenyl]-5-methyl-6-(trifluoromethyl)-5,6-dihydro-2H-1,4-oxazin-3-amine;
(5*R*,6*R*)-6-fluoro-5-{2-fluoro-5-[(3-methoxypyrazin-2-yl)amino]phenyl}-5-methyl-5,6-dihydro-2*H*-1,4-oxazin-3-amine; and
(5*R*,6*R*)-5-{2-fluoro-5-[(3-methoxypyrazin-2-yl)amino]phenyl}-5-methyl-6-(trifluoromethyl)-5,6-dihydro-2*H*-1,4-oxazin-3-amine.

**6.** A pharmaceutical composition comprising a therapeutically effective amount of a compound as defined in any one of claims 1 to 5 and a pharmaceutically acceptable carrier.

**7.** A process for preparing a pharmaceutical composition as defined in claim 6, **characterized in that** a pharmaceutically acceptable carrier is intimately mixed with a therapeutically effective amount of a compound as defined in any one of claims 1 to 5.

8. A compound as defined in any one of claims 1 to 5 for use in the treatment or prevention of Alzheimer's disease (AD), mild cognitive impairment, senility, dementia, dementia with Lewy bodies, cerebral amyloid angiopathy, multi-infarct dementia, Down's syndrome, dementia associated with stroke, dementia associated with Parkinson's disease or dementia associated with beta-amyloid.

**Patentansprüche**

1. Verbindung der Formel (I)

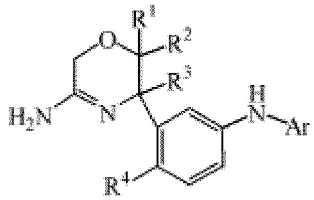

oder ein Tautomer oder eine stereoisomere Form davon, wobei

$R^1$ für Fluor, Fluormethyl, Difluormethyl oder Trifluormethyl steht,
$R^2$ für Wasserstoff oder Trifluormethyl steht oder
$R^1$ und $R^2$ einen zweiwertigen Rest $=CF_2$ bilden,
$R^3$ für Wasserstoff, $C_1$-$C_3$-Alkyl, Cyclopropyl, Mono- oder Polyhalogen-$C_1$-$C_3$-alkyl steht,
$R^4$ für Wasserstoff oder Fluor steht,
Ar für Homoaryl oder Heteroaryl steht,
wobei Homoaryl für Phenyl oder durch einen, zwei oder drei aus der aus Halogen, Cyano, $C_1$-$C_3$-Alkyl, $C_1$-$C_3$-Alkyloxy, Mono- und Polyhalogen-$C_1$-$C_3$-alkyl und Mono- und Polyhalogen-$C_1$-$C_3$-alkyloxy bestehenden Gruppe ausgewählte Substituenten substituiertes Phenyl steht,
Heteroaryl aus der aus Pyridyl, Pyrimidyl, Pyrazyl, Pyridazyl, Furanyl, Thienyl, Pyrrolyl, Pyrazolyl, Imidazolyl, Triazolyl, Tetrazolyl, Thiazolyl, Isothiazolyl, Thiadiazolyl, Oxazolyl, Isoxazolyl und Oxadiazolyl bestehenden Gruppe ausgewählt ist, jeweils gegebenenfalls substituiert durch einen, zwei oder drei aus der aus Halogen, Cyano, $C_1$-$C_3$-Alkyl, $C_2$-$C_3$-Alkinyl, $C_1$-$C_3$-Alkyloxy, Mono- und Polyhalogen-$C_1$-$C_3$-alkyl, Mono- und Polyhalogen-$C_1$-$C_3$-alkyloxy und $C_1$-$C_3$-Alkyloxy-$C_1$-$C_3$-alkyloxy bestehenden Gruppe ausgewählte Substituenten,
oder ein Additionssalz davon.

2. Verbindung nach Anspruch 1, wobei

$R^1$ für Fluor, Difluormethyl oder Trifluormethyl steht,
$R^2$ für Wasserstoff oder Trifluormethyl steht oder
$R^1$ und $R^2$ einen zweiwertigen Rest $=CF_2$ bilden,
$R^3$ für $C_1$-$C_3$-Alkyl, Cyclopropyl, Mono- oder Polyhalogen-$C_1$-$C_3$-alkyl steht,
$R^4$ für Wasserstoff oder Fluor steht,
Ar für Heteroaryl steht,
wobei Heteroaryl für Pyridyl oder Pyrimidyl steht, jeweils gegebenenfalls substituiert durch einen, zwei oder drei aus der aus Halogen, Cyano, $C_1$-$C_3$-Alkyl, $C_2$-$C_3$-Alkinyl, $C_1$-$C_3$-Alkyloxy, Mono- und Polyhalogen-$C_1$-$C_3$-alkyl, Mono- und Polyhalogen-$C_1$-$C_3$-alkyloxy und $C_1$-$C_3$-Alkyloxy-$C_1$-$C_3$-alkyloxy bestehenden Gruppe ausgewählte Substituenten, oder ein Additionssalz davon.

3. Verbindung nach Anspruch 1, wobei

$R^1$ für Fluor, Difluormethyl oder Trifluormethyl steht und $R^2$ für Wasserstoff steht oder
$R^1$ für Fluor steht und $R^2$ für Trifluormethyl steht oder
$R^1$ und $R^2$ einen zweiwertigen Rest $=CF_2$ bilden,
$R^3$ für Methyl oder Cyclopropyl steht,
$R^4$ für Wasserstoff oder Fluor steht,
Ar für Heteroaryl steht,
wobei Heteroaryl für durch Methoxy substituiertes Pyridyl oder für Pyrimidyl steht,

oder ein Additionssalz davon.

**4.** Verbindung nach Anspruch 1, wobei das durch $R^3$ substituierte Kohlenstoffatom die R-Konfiguration aufweist.

**5.** Verbindung nach Anspruch 1, ausgewählt aus

(5*R*,6*S*)-5-Cyclopropyl-6-fluor-5-{3-[(3-methoxypyridin-2-yl)amino]phenyl}-6-(trifluormethyl)-5,6-dihydro-2H-1,4-oxazin-3-amin,
(5*R*,6*R*)-6-Fluor-5-{2-fluor-5-[(3-methoxypyridin-2-yl)amino]phenyl}-5-methyl-5,6-dihydro-2H-1,4-oxazin-3-amin,
(5*R*)-6-(Difluormethyliden)-5-{2-fluor-5-[(3-methoxypyridin-2-yl)amino]phenyl}-5-methyl-5,6-dihydro-2H-1,4-oxazin-3-amin,
(5*R*,6*R**)-6-(Difluormethyl)-5-{2-fluor-5-[(3-methoxypyridin-2-yl)amino]phenyl}-5-methyl-5,6-dihydro-2H-1,4-oxazin-3-amin,
(5*R*,6*R*)-6-Fluor-5-[2-fluor-5-(pyrimidin-2-ylamino)phenyl]-5-methyl-5,6-dihydro-2H-1,4-oxazin-3-amin,
(5*R*,6*R*)-5-{2-Fluor-5-[(3-methoxypyridin-2-yl)amino]phenyl}-5-methyl-6-(trifluormethyl)-5,6-dihydro-2H-1,4-oxazin-3-amin,
(5*R*,6*R*)-5-[2-Fluor-5-(pyrimidin-2-ylamino)phenyl]-5-methyl-6-(trifluormethyl)-5,6-dihydro-2H-1,4-oxazin-3-amin,
(5*R*,6*R*)-6-Fluor-5-{2-fluor-5-[(3-methoxypyrazin-2-yl)amino]phenyl}-5-methyl-5,6-dihydro-2*H*-1,4-oxazin-3-amin und
(5*R*,6*R*)-5-{2-Fluor-5-[(3-methoxypyrazin-2-yl)amino]phenyl}-5-methyl-6-(trifluormethyl)-5,6-dihydro-2*H*-1,4-oxazin-3-amin.

**6.** Pharmazeutische Zusammensetzung, umfassend eine therapeutisch wirksame Menge einer wie in einem der Ansprüche 1 bis 5 definierten Verbindung und einen pharmazeutisch unbedenklichen Träger.

**7.** Verfahren zur Herstellung einer wie in Anspruch 6 definierten pharmazeutischen Zusammensetzung, **dadurch gekennzeichnet, dass** man einen pharmazeutisch unbedenklichen Träger innig mit einer therapeutisch wirksamen Menge einer wie in einem der Ansprüche 1 bis 5 definierten Verbindung mischt.

**8.** Verbindung, definiert wie in einem der Ansprüche 1 bis 5, zur Verwendung bei der Behandlung oder Prävention von Alzheimer-Krankheit (AD), milder kognitiver Störung, Senilität, Demenz, Demenz mit Lewy-Körperchen, zerebraler Amyloidangiopathie, Multiinfarktdemenz, Down-Syndrom, mit Schlaganfall assoziierter Demenz, mit Parkinson-Krankheit assoziierter Demenz oder mit beta-Amyloid assoziierter Demenz.

## Revendications

**1.** Composé de Formule (I)

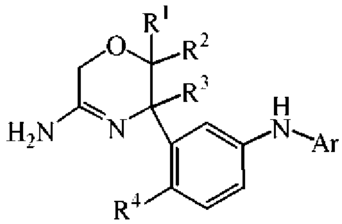

ou l'une des formes tautomères ou stéréoisomères de celui-ci, où

$R^1$ représente un groupement fluoro, fluorométhyle, difluorométhyle ou trifluorométhyle ;
$R^2$ représente un atome d'hydrogène ou un groupement trifluorométhyle ; ou
$R^1$ et $R^2$ forment un radical divalent $=CF_2$ ;
$R^3$ représente un atome d'hydrogène ou un groupement alkyle en $C_{1-3}$, cyclopropyle, mono- ou polyhalogénoalkyle en $C_{1-3}$ ;
$R^4$ représente un atome d'hydrogène ou un groupement fluoro ;

Ar représente un groupement homoaryle ou hétéroaryle ; où « homoaryle » représente un groupement phényle ou phényle substitué par un, deux ou trois substituants choisis dans le groupe constitué par les groupements halogéno, cyano, alkyle en $C_{1-3}$, alkoxy en $C_{1-3}$, mono- et polyhalogénoalkyle en $C_{1-3}$, mono- et polyhalogénoalkoxy en $C_{1-3}$ ;
le groupement hétéroaryle est choisi dans le groupe constitué par les groupements pyridyle, pyrimidyle, pyrazyle, pyridazyle, furanyle, thiényle, pyrrolyle, pyrazolyle, imidazolyle, triazolyle, tétrazolyle, thiazolyle, isothiazolyle, thiadiazolyle, oxazolyle, isoxazolyle et oxadiazolyle, chacun étant éventuellement substitué par un, deux ou trois substituants choisis dans le groupe constitué par les groupements halogéno, cyano, alkyle en $C_{1-3}$, alcynyle en $C_{2-3}$, alkoxy en $C_{1-3}$, mono- et polyhalogénoalkyle en $C_{1-3}$, mono- et polyhalogénoalkoxy en $C_{1-3}$, et (alkoxy en $C_{1-3}$)-(alkoxy en $C_{1-3}$) ;

ou l'un des sels d'addition de celui-ci.

**2.** Composé selon la revendication 1, où

$R^1$ représente un groupement fluoro, difluorométhyle ou trifluorométhyle ;
$R^2$ représente un atome d'hydrogène ou un groupement trifluorométhyle ; ou
$R^1$ et $R^2$ forment un radical divalent $=CF_2$ ;
$R^3$ représente un groupement alkyle en $C_{1-3}$, cyclopropyle, mono- ou polyhalogénoalkyle en $C_{1-3}$ ;
$R^4$ représente un atome d'hydrogène ou un groupement fluoro ;
Ar représente un groupement hétéroaryle ;
où le groupement hétéroaryle représente un groupement pyridyle ou pyrimidyle, chacun étant éventuellement substitué par un, deux ou trois substituants choisis dans le groupe constitué par les groupements halogéno, cyano, alkyle en $C_{1-3}$, alcynyle en $C_{2-3}$, alkoxy en $C_{1-3}$, mono- et polyhalogénoalkyle en $C_{1-3}$, mono- et polyhalogénoalkoxy en $C_{1-3}$, et (alkoxy en $C_{1-3}$)-(alkoxy en $C_{1-3}$) ;
ou l'un des sels d'addition de celui-ci.

**3.** Composé selon la revendication 1, où

$R^1$ représente un groupement fluoro, difluorométhyle ou trifluorométhyle, et $R^2$ représente un atome d'hydrogène ; ou
$R^1$ représente un groupement fluoro et $R^2$ représente un groupement trifluorométhyle ; ou
$R^1$ et $R^2$ forment un radical divalent $=CF_2$ ;
$R^3$ représente un groupement méthyle ou cyclopropyle ;
$R^4$ représente un atome d'hydrogène ou un groupement fluoro ;
Ar représente un groupement hétéroaryle ;
où le groupement hétéroaryle représente un groupement pyridyle substitué par méthoxy ou pyrimidyle ;

ou l'un des sels d'addition de celui-ci.

**4.** Composé selon la revendication 1, où l'atome de carbone substitué par $R^3$ présente la configuration R.

**5.** Composé selon la revendication 1, choisi parmi les suivantes :

(5R,6S)-5-cyclopropyl-6-fluoro-5-{3-[(3-méthoxypyridin-2-yl)amino]phényl}-6-(trifluorométhyl)-5,6-dihydro-2H-1,4-oxazin-3-amine ;
(5R,6R)-6-fluoro-5-{2-fluoro-5-[(3-méthoxypyridin-2-yl)amino]phényl}-5-méthyl-5,6-dihydro-2H-1,4-oxazin-3-amine,
(5R)-6-(difluorométhylidène)-5-{2-fluoro-5-[(3-méthoxypyridin-2-yl)amino]phényl}-5-méthyl-5,6-dihydro-2H-1,4-oxazin-3-amine ;
(5R,6R*)-6-(Difluorométhyl)-5-{2-fluoro-5-[(3-méthoxypyridin-2-yl)amino]phényl}-5-méthyl-5,6-dihydro-2H-1,4-oxazin-3-amine ;
(5R,6R)-6-fluoro-5-[2-fluoro-5-(pyrimidin-2-ylamino)phényl]-5-méthyl-5,6-dihydro-2H-1,4-oxazin-3-amine ;
(5R,6R)-5-{2-fluoro-5-[(3-méthoxypyridin-2-yl)amino]phényl}-5-méthyl-6-(trifluorométhyl)-5,6-dihydro-2H-1,4-oxazin-3-amine ;
(5R,6R)-5-[2-fluoro-5-(pyrimidin-2-ylamino)phényl]-5-méthyl-6-(trifluorométhyl)-5,6-dihydro-2H-1,4-oxazin-3-amine ;
(5R,6R)-6-fluoro-5-{2-fluoro-5-[(3-méthoxypyrazin-2-yl)amino]phényl}-5-méthyl-5,6-dihydro-2H-1,4-oxazin-3-amine ; et
(5R,6R)-5-{2-fluoro-5-[(3-méthoxypyrazin-2-yl)amino]phényl}-5-méthyl-6-(trifluorométhyl)-5,6-dihydro-2H-1,4-oxazin-3-amine.

**6.** Composition pharmaceutique comprenant une quantité thérapeutiquement active d'un composé selon l'une quelconque des revendications 1 à 5 ainsi qu'un vecteur pharmaceutiquement acceptable.

**7.** Procédé d'élaboration d'une composition pharmaceutique selon la revendication 6, **caractérisé en ce qu'**un vecteur pharmaceutiquement acceptable est intimement mélangé à une quantité thérapeutiquement active d'un composé selon l'une quelconque des revendications 1 à 5.

**8.** Composé selon l'une quelconque des revendications 1 à 5, pour utilisation dans le traitement prophylactique ou thérapeutique de la maladie d'Alzheimer, d'une lésion cérébrale traumatique, du déficit cognitif léger, de la sénilité, de la démence, de la démence à corps de Lewy, de l'angiopathie amyloïde cérébrale, de la démence vasculaire, du syndrome de Down, de la démence associée aux accidents vasculaires cérébraux, de la démence associée à la maladie de Parkinson ou de la démence associée aux bêta-amyloïdes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011009943 A, Novartis
- WO 2011020806 A, Hoffmann-LaRoche

### Non-patent literature cited in the description

- International Union of Pure and Applied Chemistry (IUPAC). Advanced Chemical Development, Inc, October 2006